# EUROPEAN PATENT APPLICATION

(11) **EP 4 806 913 A1**
(43) Date of publication of application: **16.09.2026**
(21) Application number: 24889151.7
(22) Date of filing: 07.11.2024
(51) Int. Cl.: C07K 14/78, C07K 16/28, C07K 14/725, C07K 14/705, C12N 5/0783, A61K 39/00, A61P 35/00

(54) **HYDROPHILIC MONOBODY HAVING IMPROVED PROPERTIES TARGETING EPHA2 AND MULTISPECIFIC CHIMERIC ANTIGEN RECEPTOR AND ENGINEERED IMMUNE CELL COMPRISING SAME**

(30) Priority: 07.11.2023 KR 20230152496
(71) Applicant: Vaxcell-Bio Co., Ltd., Jeollanam-do 58141 (KR)
(72) Inventor: LEE, Je Jung, Gwangju 61513 (KR)
(74) Representative: Leonard, Thomas Charles
(86) International application number: PCT/KR2024/017519
(87) International publication number: WO 2025/100965

(57) **Abstract**

The present invention relates to a monobody-based chimeric antigen receptor. The monobody-based chimeric antigen receptor comprises an extracellular ligand binding domain including a monobody. In addition, the present invention relates to an immune cell having the monobody-based chimeric antigen receptor expressed on the cell surface membrane thereof. The monobody-based CAR-immune cell: 1) can exhibit excellent effects in preventing or treating cancer by targeting a receptor expressed on the surface of a cancer cell; 2) uses human FN3-based monobodies as extracellular ligand binding domain unlike animal-derived scFv-based CAR-immune cells, thereby exhibiting low immunogenicity when administered to humans; 3) has a smaller size than scFv, thereby providing higher tissue penetration capability; 4) can minimize side effects (e.g., occurrence of autoimmune diseases due to non-specific binding) associated with conventional antibody therapies for cancer treatment; and 5) enables various monobody-based CAR-immune cells to be produced by using an already-established monobody libraries for various cancer cell antigens. Accordingly, the present invention can be usefully employed in preventing or treating intractable solid tumors having various antigens.

## Description

### [Technical Field]

The present invention relates to a hydrophilic monobody in which a partial sequence of a conventional fibronectin(FN3) targeting EphA2 is substituted with hydrophilic amino acids, and a chimeric antigen receptor comprising the same, and more particularly, to a bispecific chimeric antigen receptor targeting both EphA2 and PD-L1. The present invention also relates to an isolated immune cell comprising the bispecific chimeric antigen receptor and its use for the treatment of cancer.

### [Background Art]

In the treatment of solid tumors, surgical resection alone has limitations in completely eliminating cancer stem cells, residual cancer cells, or metastatic cancer cells. Therefore, combination therapies that integrate surgical resection with radiation therapy, chemotherapy, target therapy, and immunotherapy are being utilized. In particular, immunotherapy utilizes the body's own immune system, thereby exhibiting few of the side effects typically associated with radiation therapy or chemotherapy.

As a representative example of immune cells, T cells are immune cells that mediate adaptive immunity and account for 75% of total lymphocytes. While most of T cells exist in an inactive state within the blood, they transform into an active form upon receiving antigenic stimulation, enabling them to eliminate cells bearing specific antigens. The action of T cells can be activated by receptors capable of recognizing antigens (T cell receptor; TCR), co-stimulatory molecules, and cytokines.

Immune cells including T cells kill cancer cells by binding to major histocompatibility complex (MHC) molecules to which tumor-specific antigens are bound; however, cancer cells inhibit the function of immune cells and achieve immune evasion through mechanisms such as suppressing the expression of MHC, altering the functions of immune checkpoints expressed on the surface of immune cells, such as CTLA-4(cytotoxic T-lymphocyte associated protein-4), PD-1(programmed cell death protein-1), LAG-3(lymphocyte-activation gene-3), KIR(killer cell immunoglobulin-like receptor), TIM-3(T-cell immunoglobulin and mucin-domain containing-3), VISTA(V-domain Ig suppressor of T cell activation), or expressing ligands(Programmed cell death protein ligand-1) for immune checkpoints. Furthermore, they alter the tumor microenvironment to create conditions favorable for tumor growth and intravascular migration. Notably, one of the greatest challenges in treating solid tumors is the presence of diverse antigens; unlike hematologic cancers, solid tumors differentiate into heterogeneous cancer cells even within the same tumor type, making it difficult to characterize the cancer. Consequently, therapies targeting at least two or more antigens have begun to be developed for the treatment of solid tumors. Examples include Catumaxomab(peritoneal cancer, withdrawn from the market in 2017), which was approved as the first bispecific antibody in 2009, Blincyto(hematologic cancer), which was the second to be approved in 2014, and recently Kimmtrak and Lunsumio, which were approved in the United States and Europe in 2022.

As it has been revealed that cancer cells can survive from immune cell attacks by utilizing immune checkpoints, the development of drugs that interfere with the binding between immune checkpoint proteins and ligands on cancer cells has been actively pursued. As a result, monoclonal antibodies capable of specifically binding to immune checkpoint proteins, such as PD-1/CTLA-4, have been developed. These PD-1 or CTLA-4 antibodies bind to the PD-1 and CTLA-4 proteins present on the immune cell membrane, respectively, preventing these checkpoint proteins from binding to their ligands present on cancer cells. This allows the cancer cell-killing function of the immune cells to be maintained. Examples of such antibody-based cancer immunotherapies include Atezolizumab, Avelumab, and Ipilimumab.

The ephrin receptor, which is a representative cancer antigen but for which no antibody therapeutic has yet been developed, is a membrane receptor tyrosine kinase (Receptor Tyrosine Kinase, RTK) with a size of 108kDa and is divided into three parts: an extracellular portion comprising a ligand-binding domain, a cysteine-rich region, and two fibronectin type III repeat units; a transmembrane portion in the middle; and a cytoplasmic portion having a kinase activity domain (Labrador et al., 1997; Pasquale, 1997). Ephrin receptors bind to their ligands, ephrins, to transduce extracellular signals into the cell via phosphorylation and are involved in cell proliferation, migration, differentiation, synaptic transmission in neurons, tissue remodeling, osteocyte differentiation, and angiogenesis (Pasquale EB. Cell. 133:38-52, 2008; Barquilla A et al., Annu. Rev. Pharmacol. Toxicol. 55:465-487, 2015). Ephrin receptors are classified into 9 types of Group A (EphA1-8 and EphA10) and 5 types of Group B (EphB1-4 and EphB6), based on structure of the receptor and ligand-receptor binding specificity (Ieguchi K. Endocr Metab Immune Disord Drug Targets. 15:119-128, 2015; Eph Nomenclature Committee. Cell. 90:403-404, 1997; Lindberg RA et al., Mol Cell Biol. 10(12):6316-6324, 1990; Davis S et al., Science. 266(5186):816-819, 1994). Among these, Ephrin receptor A2(EphA2) is expressed in extremely small amounts in normal cells but is non-specifically overexpressed in most cancer cells, including liver, prostate, pancreatic, head and neck, gastric, colon, lung, cervical, and ovarian cancers so that it promotes carcinogenesis, metastasis, angiogenesis, and resistance of tumors. EphA2 shares approximately 65% protein homology with EphA1 and is found in nearly identical locations in mouse lungs. It is overexpressed along with EphA1 and their ligand, Ephrin-A1, in various malignant tumors, including non-small cell lung cancer (NSCLC). Through interactions with the EphA2/A1-Ephrin-A1 complex, it contributes to oncogenesis and malignant progression (Naj AC et al., Nat Genet. 43:436-441, 2011; Finney AC et al., Circulation. 136:566-582, 2017).

Since 2017, immune cell-based cancer immunotherapies have been developed as a method to eliminate cancer cells using the body's immune cells by specifically binding to them, distinguishing these from conventional immunotherapies. These include T cells expressing, in cells, chimeric antigen receptors(CAR) (CAR T cells) capable of directly recognizing tumor-associated antigens, or T cells expressing antibody sequences specific to immune checkpoint receptors of cancer cells. These cancer therapeutics are designed to enable immune cells to specifically attack cancer cells.

The structure of currently developed chimeric antigen receptors is divided into a single chain variable fragment (scFv) part that recognizes antigens, a transmembrane domain, and a signaling domain that delivers signals into the cell. Pharmaceutical companies including Novartis, Gilead, Celgene, AbClon, and Janssen, and clinicians have developed CAR-T using scFv that specifically binds to the CD19 antigen of hematologic cancer cells as a treatment for hematologic cancer or using nanobody that specifically binds to BCMA, an antigen of multiple myeloma.

CAR-based immune cell therapy integrated with various genetic engineering technologies is absolutely required for solid tumors, which constitute most cancers. However, in the case of solid tumors, there are limitations in heterogeneity where patients express different antigens, and complex characteristics of the tumor microenvironment, such as hypoxia, as well as T cell migration and activity. According to recent clinical trial reports, when dual targets are used to overcome heterogeneity, a stronger immune response is induced compared to targeting a single antigen, leading to reports of serious side effects, including cytokine storms and neurotoxicity. Meanwhile, in cases where antibodies used in antibody drugs are utilized as they are-specifically, when the antigen-recognition site is used for CAR immune cell therapy without considering the strong binding characteristics with the antigen inherent to antibody drugs-serious side effects such as on-target, off-tumor toxicity are being reported.

Conventional cancer immunotherapy is known as a treatment for hematologic cancer in which mouse-based antibody molecules, specifically antibody fragments (scFv), recognize and bind to human cancer antigens, followed by the elimination of cancer cells through the secretion of various cytokines or attacks by surrounding immune cells; however, it has limitations such as side effects caused by animal-based antibodies, low penetration into tissues due to the size of scFv, and the requirement to simultaneously recognize at least two or more antigens due to the characteristics of solid tumors where different antigens are expressed in each patient (heterogeneity).

While conducting research to compensate for the disadvantages of scFv and the disadvantages of hydrophobic monobodies, the present inventors confirmed that using a hydrophilic monobody, based on PD-L1 scFv and hydrophilic human fibronectin FN3, as the extracellular ligand-binding domain of a chimeric antigen receptor provides excellent effects in preventing and treating solid tumors, and thus completed the present invention.

### (Prior Art Documents)

### (Patent Documents)

(Patent Document 1) Korean Registered Patent No. 10-2533540
(Patent Document 2) Korean Registered Patent No. 10-2048477
(Patent Document 3) Korean Registered Patent No. 10-2157197

### [Summary of Invention]

### [Technical Problem]

An object of the present invention is to provide a hydrophilic monobody that specifically binds to EphA2.

An object of the present invention is to provide a chimeric antigen receptor comprising said monobody.

An object of the present invention is to provide a vector comprising a polynucleotide containing a nucleic acid sequence encoding said monobody or said chimeric antigen receptor.

An object of the present invention is to provide said polynucleotide.

An object of the present invention is to provide an immune cell expressing an extracellular ligand-binding domain comprising said monobody or the chimeric antigen receptor on its cell surface membrane.

An object of the present invention is to provide a pharmaceutical composition for preventing or treating cancer, comprising said immune cell.

### [Technical Solution]

The present invention provides a hydrophilic monobody in which one or more polar non-ionic amino acids of a monobody consisting of the amino acid sequence of SEQ ID NO: 46 are substituted with hydrophobic amino acids. Said monobody may comprise the amino acid sequence of SEQ ID NO: 8.

Said monobody may be linked to a PD-L1 scFv directly or through a linker. Said PD-L1 scFv may comprise the amino acid sequence of SEQ ID NO: 27.

The present invention provides a chimeric antigen receptor comprising said monobody as an extracellular ligand-binding domain, and further comprising one or more selected from the group consisting of a transmembrane domain and an intracellular signaling domain.

Said transmembrane domain may be selected from the group consisting of T-cell receptor alpha chain, T-cell receptor beta chain, T-cell receptor zeta chain, CD8 alpha chain, CD8 beta chain, CD28, CD3 epsilon, CD45, CD4, CD5, CD8, CD9, CD16, CD22, CD33, CD37, CD64, CD80, CD86, CD134, CD137, CD154, a part thereof, and any combination thereof.

Said intracellular signaling domain may be selected from the group consisting of TCR zeta, FcR gamma, FcR beta, FcR epsilon, CD3 gamma, CD3 delta, CD3 epsilon, CD3 zeta, CD5, CD22, CD79a, CD79b, and CD66d, and any combination thereof.

Said intracellular signaling domain may be one or more selected from the group consisting of OX40 domain, CD2 domain, CD27 domain, CD28 domain, CDS domain, ICAM-1 domain, LFA-1 domain, and 4-1BB domain, and any combination thereof.

Said chimeric antigen receptor may further comprise a hinge.

The present invention provides a polynucleotide comprising a nucleic acid sequence encoding said monobody or said chimeric antigen receptor.

The present invention provides a vector comprising said polynucleotide.

The present invention provides an immune cell expressing, on its cell surface membrane, an extracellular ligand-binding domain comprising said monobody or said chimeric antigen receptor.

Said immune cell may be selected from the group consisting of leukocytes, neutrophils, eosinophils, basophils, monocytes, lymphocytes, T cells, cytotoxic T cells, natural killer T cells, and dendritic cells.

The present invention provides a pharmaceutical composition for preventing or treating cancer, comprising said immune cell.

Said cancer may be selected from the group consisting of melanoma, squamous cell carcinoma, breast cancer, head and neck cancer, thyroid cancer, soft tissue sarcoma, osteosarcoma, testicular cancer, prostate cancer, ovarian cancer, bladder cancer, skin cancer, brain cancer, angiosarcoma, mastocytoma, leukemia, lymphoma, liver cancer, lung cancer, pancreatic cancer, gastric cancer, kidney cancer, colorectal cancer, hematopoietic tumor, and metastatic cancer thereof.

Said cancer may be pancreatic cancer, prostate cancer, or ovarian cancer.

### [Advantageous Effects of Invention]

The hydrophilic monobody-based chimeric antigen receptor according to the present invention, an immune cell expressing the same on its cell surface membrane, and a composition for preventing or treating cancer using the same can exhibit the following effects.
1) Side effects(e.g., hair loss and digestive disorders) caused by inhibiting even the growth of normal cells in conventional radiation therapy or chemotherapy using DNA nucleotide analogs(cisplatin-based and 5-FU-based), which only inhibit rapid cell proliferation, can be reduced.
2) Since a small-sized monobody based on human FN3 is used, it is possible to minimize cancer cell metastasis caused by resistance to anticancer agents and side effects of autoimmune diseases, which occur in targeted antibody therapies used as animal-derived antibody-based anticancer therapies and chimeric T-cell therapies using scFv obtained from libraries of mouse monoclonal antibodies.
3) It is possible to simultaneously diagnose and treat cancer, as it can effectively inhibit or eliminate solid tumors expressing a cancer antigen(e.g., EphA2), overcoming the limitations of monobodies that have been used only for diagnostic purposes due to their lack of cancer-killing ability, instability, and short lifespan.
4) Monobody-based CAR-immune cells can exhibit superior anticancer effects compared to conventional treatments for solid tumors where T-cells are administered alone, and can be used as a cancer immunotherapy to treat or prevent solid tumors such as pancreatic cancer, prostate cancer, and ovarian cancer that express EphA2 as an antigen, for example.
5) FN3 monobodies have the advantage of being able to produce various types of monobodies against target antigens. Accordingly, it is possible to produce monobody-based CAR-immune cells specific to various antigens, which is very effective in treating cancer.
6) Since monobody-based CAR-immune cells are smaller in size than other CAR-immune cells(for example, the size of a monobody is about one-third the size of an scFv), penetration into cancer tissues is easier than with scFv-based CARs; furthermore, when producing a multivalent CAR in a viral vector, up to four monobody sequences recognizing cancer antigens can be loaded into the vector considering viral packing, which is advantageous in overcoming the heterogeneity of cancer antigens, a characteristic of solid tumors. In addition, since it has sufficient selectivity and specificity for cancer antigens, it can block immune cell evasion of cancer and effectively prevent or treat cancer.
7) While conventional hydrophilic fibronectin FN3 domains have been reported in research aimed at supplementing their function as biomarkers by enabling strong binding to targets and absorption into intracellular endosomes (Sirois AR, Deny DA, Baierl SR, George KS, Moore SJ (2018) Fn3 proteins engineered to recognize tumor biomarker mesothelin internalize upon binding. PLoS ONE 13(5): e0197029. https:// doi.org/10.1371/journal.pone.0197029), there have been no reports of their use for therapeutic purposes, particularly as cell and gene therapies. It was confirmed that the hpEphA2 monobody-based CAR-immune cells (hpVEC) using the hydrophilic fibronectin FN3 domain, for which a patent application is to be filed, exhibit an expression rate more than twice as high as that of conventional EphA2 monobody CAR-immune cells (VEC), which means that the probability of recognizing target cancer cells in the body can be much higher. Furthermore, immunogenicity could be further reduced during in silico immunogenicity prediction analysis.

The chimeric antigen receptor specific to two cancer antigens, PD-L1 and EphA2, and the immune cell comprising the same according to the present invention not only exhibit excellent targeting efficiency specific to PD-L1 and EphA2, but also show superior anticancer effects even against cancer cells expressing only a single antigen of PD-L1 or EphA2, even if the cancer cells do not express or spontaneously eliminate PD-L1 or EphA2 for immune evasion; therefore, they can be usefully employed for preventing or treating tumor growth and metastasis.

### [Brief Description of Drawings]

FIG. 1 is a schematic diagram showing the structure of a vector expressing a hydrophilic monobody-based chimeric antigen receptor.
FIG. 2a is a schematic diagram comparing the amino acid sequences of a non-hydrophilic monobody(E1) and a hydrophilic monobody(hpE1).
FIG. 2b is a schematic diagram showing the structural similarity by superimposing the tertiary structures of a non-hydrophilic monobody(E1)-based and a hydrophilic monobody(hpE1)-based chimeric antigen receptors.
FIG. 2c is a graph comparing the protein binding affinity for the human antigen EphA2 between a non-hydrophilic monobody(E1) and a hydrophilic monobody(hpE1).
FIG. 3 is a graph comparing the transduction efficiency between T-immune cells(hpVEC-1) comprising a hydrophilic monobody-based chimeric antigen receptor and T-immune cells(VEC-1) comprising a non-hydrophilic monobody-based chimeric antigen receptor.
FIG. 4 is a graph comparing activation markers between hpVEC-1 and VEC-1.
FIGs. 5a and 5b are graphs comparing inhibitory markers between hpVEC-1 and VEC-1.
FIG. 6 is a graph comparing the cytotoxicity of hpVEC-1 and VEC-1 against a HEK293 kidney cell line overexpressing the human cancer antigen EphA2.
FIGS. 7a and 7b are graphs comparing the in vitro cytotoxicity of hpVEC-1 and VEC-1 against cancer cells(ovarian cancer, gastric cancer, glioblastoma, and pancreatic cancer) using the RTCA method.
FIG. 8 is a graph showing the results of FACS analysis on the characteristics of immune cells 24 days after the completion of production of hpVEC-1 and VEC-1, specifically the expression levels of activation factors CD62L, CD25, and CD69 and inhibitory factors CTLA-4, TIGIT, TIM-3, LAG-3, and PD-1.
FIG. 9 is a graph showing the cancer cell-killing ability of hpVEC-1 and VEC-1 immune cells using the RTCA analysis method.
FIG. 10 is a graph showing the cancer cell-killing ability of hpVEC-1 and VEC-1 against ovarian and gastric cancer cells over time, using a 3D spheroid co-culture method and an ELISA reader measuring the amount of LDH released from dead cancer cells in the medium.
FIG. 11 is a graph showing changes in tumor size and mouse body weight recovery in an ovarian cancer xenograft mouse model.
FIG. 12 is a schematic diagram illustrating the structure of a dual chimeric antigen receptor vector that simultaneously recognizes two antigens, PD-L1 and EphA2.
FIG. 13 is a graph comparing activation markers and inhibitory markers, which are characteristics of T-lymphocyte immune cells comprising heterogeneous chimeric antigen receptors.
FIG. 14 is a graph comparing the cytotoxicity of T-lymphocyte immune cells comprising PD-L1 and EphA2 dual antigen-recognizing chimeric antigen receptors against HEK293 cell lines overexpressing PD-L1, EphA2, or both antigens simultaneously, using the RTCA method.
FIG. 15 is a graph comparing the cytotoxicity of T-lymphocyte immune cells comprising PD-L1 and EphA2 dual antigen-recognizing chimeric antigen receptors against two types of subpopulations within the gastric cancer-derived ovarian cancer cell line OVCAR-5, using the RTCA method.
FIG. 16 is a graph showing changes in tumor size and mouse body weight recovery in an ovarian cancer xenograft mouse model.
FIG. 17 is a graph showing mouse survival curves plotted using the Kaplan-Meier method in an ovarian cancer xenograft mouse model.

### [Best Mode for Carrying Out the Invention]

Throughout the entire specification of the present application, when a certain part "includes" a certain component, this means that it may further include other components, rather than excluding other components, unless otherwise specifically stated.

Unless defined in detail in the present invention, all technical and scientific terms used in the present invention may have the same meanings as commonly understood by a person of ordinary skill in the fields of gene therapy, biochemistry, genetics, and molecular biology.

Unless otherwise specified, the practice of the present invention may utilize conventional techniques of cell biology, cell culture, molecular biology, microbiology, recombinant DNA, and immunology, which are within the skill of the art.

As used in the present invention, the term "prevention" refers to any action that suppresses or delays the onset of a disease by administration of a composition; "treatment" refers to any action that improves or beneficially changes the symptoms of an individual suspected of or having a disease by administration of a composition; and "improvement" refers to any action that at least reduces parameters related to the condition, such as the severity of symptoms, by administration of a composition.

### PD-L1 scFv and Hydrophilic Monobody-Based Chimeric Antigen Receptor

The present invention relates to a PD-L1 scFv and a hydrophilic monobody-based chimeric antigen receptor (CAR).

The PD-L1 scFv and the hydrophilic monobody-based chimeric antigen receptor of the present invention may comprise an extracellular ligand-binding domain.

The term "extracellular ligand-binding domain" as used in the present invention may refer to an oligopeptide or polypeptide capable of binding a ligand. Preferably, the domain can interact with cell surface molecules. For example, the extracellular ligand-binding domain can be selected to recognize a ligand that acts as a cell surface marker on a target cell associated with any disease.

The term "Linker" as used in the present invention refers to amino acids that connect between protein domain-domain. The "Linker" of the present invention may have at least 70%, preferably at least 80%, more preferably at least 90%, 95%, 97%, or 99% sequence identity with the amino acid sequence of SEQ ID NO: 19.

The term "hinge" as used in the present invention may be a naturally occurring hinge region or a synthetic amino acid sequence, and preferably may be a part of a CD8 alpha chain.

The SEQ ID NO of the PD-L1 scFv of the present invention may have at least 70%, preferably at least 80%, more preferably at least 90%, 95%, 97%, or 99% sequence identity with the amino acid sequence of SEQ ID NO: 18.

The term "hydrophilic monobody (hp)" as used in the present invention may refer to specifically binding to a ligand or antigen present on the surface of a target cell in a stable form between proteins such as hydrogen bonding, by substituting hydrophobic amino acid sequences in the backbone of an EphA2 monobody with polar uncharged amino acids.

The hydrophilic monobody may be composed of amino acids having uncharged polar side chains. Accordingly, it may have a charge depending on pH, and the side chains of serine (S) and threonine (T) include hydroxyl groups and can form hydrogen bonds to stabilize protein structures or be important in other biochemical reactions, specifically playing an important role in protein-protein interactions or interactions between proteins and other molecules. In particular, asparagine (N) and glutamine (Q) may be important for targeting and protein recognition.

A hydrophilic monobody may be prepared by substituting hydrophobic amino acids such as glycine (G), alanine (A), valine (V), leucine (L), isoleucine (I), proline (P), methionine (M), phenylalanine (F), and tryptophan (W) with hydrophilic amino acids such as serine (S), threonine (T), tyrosine (Y), cysteine (C), asparagine (D), and glutamic acid (E), or by adding hydrophilic amino acids. For example, the hydrophilicity can be increased by substituting hydrophobic valine (V) with a hydrophilic amino acid, serine (S) or threonine (T), and hydrophobic leucine (L) with hydrophilic threonine (T), or by adding an amino acid sequence of 1 to 1,000 amino acids including hydrophilic amino acids (E, S).

A monobody may be variously selected depending on a desired target. For example, the monobody may specifically bind to a protein selected from the group consisting of CRL1, ephrin receptor, EphA2, B -galactosidase, RAS, Abl kinase, VEGFR2, MBP, SARS-CoV-2, Bcr-Abl kinase, STAT3, EGFR, VEGFR2, SH3 domain of human Lyn tyrosine kinase, MLKL, Fluc homologues, mitogen-activated protein kinase (MAPK), ERK2, MAPK14, kinase SH2 domain, SUMO, AurA, WDR5, Fluc family, GFP, receptor-binding domain of SARS-CoV-2, SH3 domain of FYN, SH2 domain of ABL, SUMO1, Bcr-Abl, GPR56 ECR, PD-L1, glypican-3, PCSK9, Gp41, CD4, and IL-23, but is not limited thereto.

Also, the monobody may be one known in the art.

The monobody may specifically bind to an ephrin receptor, EphA2, or human EphA2.

The monobody may have at least 70%, preferably at least 80%, more preferably at least 90%, 95%, 97%, or 99% sequence identity with the amino acid sequence of SEQ ID NO: 8.

A monobody-based CAR may further include one or more selected from the group consisting of a hinge, a transmembrane domain, and an intracellular signaling domain.

In addition, the monobody-based CAR is expressed on the surface membrane of a cell. Accordingly, the monobody-based CAR may include a transmembrane domain. Distinguishing features of a suitable transmembrane domain include the ability to be expressed on the surface of a cell, preferably an immune cell (particularly a lymphocyte cell or an NK cell) in the present invention, and to interact together to induce a cellular response of the immune cell against a predetermined target cell. The transmembrane domain may be derived from either a natural source or a synthetic source. The transmembrane domain may be derived from any membrane-bound or transmembrane protein.

The transmembrane domain may be one or more selected from the group consisting of a T-cell receptor alpha chain, a T-cell receptor beta chain, a T-cell receptor zeta chain, a CD8 alpha chain, a CD8 beta chain, CD28, CD3 epsilon, CD45, CD4, CD5, CD8, CD9, CD16, CD22, CD33, CD37, CD64, CD80, CD86, CD134, CD137, CD154, a part thereof, and a combination thereof.

The hinge and the transmembrane domain may include a human CD8 alpha chain or a part thereof. The hinge and the transmembrane domain may include the amino acid sequence of SEQ ID NO: 15, and preferably may include an amino acid sequence having at least 70%, preferably at least 80%, more preferably at least 90%, 95%, 97%, or 99% sequence identity with the amino acid sequence of SEQ ID NO: 22.

The intracellular signaling domain of the monobody-based CAR may cause intracellular signaling after binding the extracellular ligand-binding domain to a target that triggers activation of immune cells and immune responses. The signaling domain may cause activation of at least one of the normal effector functions of the immune cell expressing the monobody-based CAR. For example, the effector function of a T cell may be cytotoxic activity or helper activity, including the secretion of cytokines.

The term "signaling domain" as used in the present invention may refer to a part of a protein that converts effector function signals within an immune cell and induces the cell to perform a specific function.

The intracellular signaling domain may include one or more selected from the group consisting of TCR zeta, FcR gamma, FcR beta, FcR epsilon, CD3 gamma, CD3 delta, CD3 epsilon, CD3 zeta, CD5, CD22, CD79a, CD79b, and CD66d.

The intracellular signaling domain may include one or more selected from the group consisting of an OX40 domain, a CD2 domain, a CD27 domain, a CD28 domain, a CDS domain, an ICAM-1 domain, an LFA-1 domain, and a 4-1BB domain.

The intracellular signaling domain may include one or more selected from the group consisting of a CD28 domain, a 4-1BB domain, and a CD3 zeta domain, and preferably, may include a CD28 domain and a CD3 zeta domain, or a 4-1BB domain and a CD3 zeta domain.

The CD28 domain may include the amino acid sequence of SEQ ID NO: 23 or an amino acid sequence having at least 70%, preferably at least 80%, more preferably at least 90%, 95%, 97%, or 99% sequence identity therewith.

The 4-1BB domain may include the amino acid sequence of SEQ ID NO: 11 or an amino acid sequence having at least 70%, preferably at least 80%, more preferably at least 90%, 95%, 97%, or 99% sequence identity therewith.

The CD3 zeta domain may include the amino acid sequence of SEQ ID NO: 12 or an amino acid sequence having at least 70%, preferably at least 80%, more preferably at least 90%, 95%, 97%, or 99% sequence identity therewith.

### Polynucleotides and Vectors

The present invention may relate to a polynucleotide comprising a nucleic acid sequence encoding a hydrophilic monobody, a CAR comprising a hydrophilic monobody and a PD-L1 scFv, or a vector comprising such a nucleic acid sequence or polynucleotide. Here, the vector may mean an expression vector.

The polynucleotide may include the nucleic acid sequence of SEQ ID NO: 2. The polynucleotide may include a nucleic acid sequence having at least 70%, preferably at least 80%, more preferably at least 90%, 95%, 97%, or 99% sequence identity with the nucleic acid sequence of SEQ ID NO: 45.

The nucleic acid sequence encoding the hydrophilic monobody-based CAR may be codon-optimized for expression in human cells.

The term "vector" as used in the present invention may refer to a construct capable of delivering, and preferably expressing, one or more gene(s) or sequence(s) of interest in a host cell. Examples include, but are not limited to, viral vectors, naked DNA or RNA expression vectors, plasmids, cosmids or phage vectors, DNA or RNA expression vectors associated with cationic condensing agents, DNA or RNA expression vectors encapsulated in liposomes, and certain eukaryotic cells, such as producer cells.

Viral vectors may include retroviruses, adenoviruses, parvoviruses (e.g., adeno-associated viruses), coronaviruses, negative-strand RNA viruses such as orthomyxoviruses (e.g., influenza virus), rhabdoviruses (e.g., rabies and vesicular stomatitis viruses), paramyxoviruses (e.g., measles and Sendai), positive-strand RNA viruses such as picornaviruses and alphaviruses, and double-stranded DNA viruses including adenoviruses, herpesviruses (e.g., herpes simplex virus types 1 and 2, Epstein-Barr virus, cytomegalovirus), and poxviruses (e.g., vaccinia, fowlpox, and canarypox). Other viruses that may be used as vectors include, for example, Norwalk virus, togaviruses, flaviviruses, reoviruses, papovaviruses, hepadnaviruses, and hepatitis viruses. Examples of retroviruses may include avian leukosis-sarcoma, mammalian C-type, B-type viruses, D-type viruses, HTLV-BLV group, lentiviruses, and spumaviruses.

### Engineered Immune Cells

The present invention may relate to an isolated cell or cell line obtainable by the method for producing or engineering said immune cell. Here, the isolated cell may comprise a monobody-based CAR. Said monobody-based CAR may preferably be a CAR comprising a hydrophilic monobody and a PD-L1 scFv according to the present invention.

The isolated cell of the present invention may comprise a population of CARs comprising different extracellular ligand-binding domains. In particular, said isolated cell may comprise an exogenous polynucleotide sequence encoding the CAR.

The immune cell of the present invention may refer to a cell of hematopoietic origin functionally involved in the initiation and/or execution of innate and/or adaptive immune responses. The immune cell of the present invention may be derived from a stem cell. The stem cell may be an adult stem cell, a non-human embryonic stem cell, a non-human stem cell, a cord blood stem cell, a progenitor cell, a bone marrow stem cell, an induced pluripotent stem cell, a totipotent stem cell, or a hematopoietic stem cell.

The immune cell of the present invention may be a human CD3+ T cell. In one embodiment, the isolated cell may be a T-cell selected from the group consisting of dendritic cells, killer dendritic cells, mast cells, NK-cells, B-cells, or inflammatory T-lymphocytes, cytotoxic T-lymphocytes, regulatory T-lymphocytes, or helper T-lymphocytes. In one embodiment, said cell may be derived from the group consisting of CD4+ T-lymphocytes and CD8+ T-lymphocytes.

The cells may be obtained from a subject through various non-limiting methods. The cells may be obtained from a number of non-limiting sources, including peripheral blood mononuclear cells, bone marrow, lymph node tissue, cord blood, thymus tissue, tissue from a site of infection, ascites, pleural effusion, spleen tissue, and tumors.

Any T cell line available and known to those skilled in the art may be used. Said cells may be derived from a healthy donor, from a patient diagnosed with cancer, or from a patient diagnosed with an infectious disease. Said cells may be part of a mixed population of cells exhibiting different phenotypic characteristics. A cell line obtained from T cells engineered according to the aforementioned method may be used, and the immune cell according to the present invention may be one that has resistance to immunosuppressive treatment.

Said immune cell may be leukocytes, neutrophils, eosinophils, basophils, monocytes, lymphocytes, T cells, cytotoxic T cells, natural killer T cells, dendritic cells, or a combination thereof, but is not limited thereto.

### Treatment Using Dual-Antigen Recognition CAR-Immune Cells Based on PD-L1 scFv and Hydrophilic Monobody

The dual-antigen recognition CAR-immune cells based on PD-L1 scFv and hydrophilic monobody, engineered immune cells, or a population of these cells may be used as a pharmaceutical composition for preventing or treating cancer.

The present invention may relate to a pharmaceutical composition for preventing or treating cancer, comprising the dual-antigen recognition CAR-immune cells based on PD-L1 scFv and hydrophilic monobody or engineered immune cells.

The present invention may relate to a method for preventing or treating cancer in a subject in need thereof, comprising administering the dual-antigen recognition CAR-immune cells based on PD-L1 scFv and hydrophilic monobody to the subject in need thereof.

The present invention may relate to the use of dual-antigen recognition CAR-immune cells based on PD-L1 scFv and hydrophilic monobody for the manufacture of a medicament for preventing or treating cancer.

The present invention may be dual-antigen recognition CAR-immune cells based on PD-L1 scFv and hydrophilic monobody for use in preventing or treating cancer.

As used in the present invention, the term "subject" is a mammal, more preferably a human. Mammals include, but are not limited to, livestock, pets, primates, horses, dogs, cats, mice, rats, and the like.

In the present invention, said treatment may be part of an autologous immunotherapy or part of an allogeneic immunotherapy regimen. Autologous may mean that the cells, cell lines, or population of cells used to treat a subject are obtained from said subject or from a donor compatible with human leukocyte antigens (HLA). Allogeneic may mean that the cells or population of cells used to treat a subject are obtained not from said subject but from a donor.

The cancer may be, but is not limited to, melanoma, squamous cell carcinoma, breast cancer, head and neck cancer, thyroid cancer, soft tissue sarcoma, osteosarcoma, testicular cancer, prostate cancer, ovarian cancer, bladder cancer, skin cancer, brain cancer, angiosarcoma, mast cell tumor, leukemia, lymphoma, liver cancer, lung cancer, pancreatic cancer, gastric cancer, kidney cancer, colorectal cancer, hematopoietic tumor, or metastatic cancer thereof. Additionally, the cancer may be a non-solid tumor or a solid cancer.

The non-solid tumor or non-solid cancer may be, but is not limited to, myeloma, lymphoma, or leukemia.

The solid cancer may be at least one selected from the group consisting of pancreatic cancer, prostate cancer, ovarian cancer, breast cancer, cervical cancer, skin cancer, colorectal cancer, kidney cancer, liver cancer, brain cancer, and lung cancer, but is not limited thereto.

Hereinafter, embodiments and examples of the present disclosure will be described in detail with reference to the accompanying drawings so that those skilled in the art to which the present invention pertains may easily practice the same. However, the present disclosure may be implemented in various forms and is not limited to the embodiments and examples described herein.

### [Example 1]

### Preparation of a Vector Expressing a Hydrophilic Monobody-Based Chimeric Antigen Receptor

### 1-1. Preparation for the Production of Recombinant Lentiviral Vectors

The following strains and vectors were prepared for the production of a recombinant plasmid vector expressing a hydrophilic monobody-based chimeric antigen receptor.

### (1) Vector to be cloned

The pMX-hpEl vector containing the hpE1 monobody nucleic acid sequence of SEQ ID NO: 2, which specifically binds to human EphA2, was synthesized by Thermo Fisher (GeneArt), and a CAR vector expressing a chimeric antigen receptor was prepared. Specifically, pLenti7.3/V5-DEST-vector from Invitrogen was used as the expression vector, and a nucleic acid sequence expressing a chimeric antigen receptor consisting of: SEQ ID NO: 1 (CD8α leader sequence) - SEQ ID NO: 45 (E1 monobody sequence) - SEQ ID NO: 3 (CD8 H&TM sequence) - SEQ ID NO: 4 (CD28 sequence) - SEQ ID NO: 6 (CD3ζ sequence); or SEQ ID NO: 1 (CD8α leader sequence) - SEQ ID NO: 45 (E1 monobody sequence) - SEQ ID NO: 3 (CD8 H&TM sequence) - SEQ ID NO: 5 (4-1BB sequence) - SEQ ID NO: 6 (CD3ζ sequence) was included between the 5' UTR and 3' UTR of said pLenti7.3/V5-DEST vector. Thus, two lentiviral vectors were prepared (pLenti7.3::CD8α leader sequence-E1 monobody sequence-CD8 H&T-CD28-CD3ζ vector and pLenti7.3::CD8α leader sequence-E1 monobody sequence-CD8 H&T-4-1BB-CD3ζ vector).

The nucleic acid sequences constituting the hydrophilic monobody-based chimeric antigen receptor in the recombinant plasmid vector are shown in Table 1 below.

**[Table 1]**

| SEQ ID NO | Sequence Name | Sequence | Remarks |
|---|---|---|---|
| 1 | CD8α signal sequence nucleotide | ATGGCCCTGCCTGTGACAG CTTTGCTGCTGCCTCTGGCT CTGCTGCTGCATGCTGCTAG ACCT | Codon-optimized nucleotide sequence of the signal sequence domain amino acid sequence (1-21, NCBI accession # NP_741969.1) of Human CD8α |
| 2 | hpE1 monomer nucleotide | AGCAGCGACAGCCCCAGAA ATCTGGAAGTGACCAACGC CACACCTAACAGCCTGACC ATCAGCTGGTACTACCCCTT CTGCGCCTTCTACTACCGGA TCACCTATGGCGAGACAGG CGGCAACAGCCCTAGCCAA GAGTTCACCGTGCCTAGAC CTAGCGACACCGCCACAAT CTCTGGCCTGAAGCCAGGC CAGGACTACACCATCACAG TGTACGCCGTGACCTGCCT GGGCAGCTACAGCAGACCC ATCAGCATCAACTACCGGA CCGAGATCGACAAGCCCAG CCAA | |
| 3 | CD8α H&TM nucleotide | ACCACCACACCAGCTCCAC GGCCTCCAACACCAGCACC TACAATTGCCTCTCAGCCCC TGTCTCTGAGGCCAGAAGC TTGTAGACCTGCTGCAGGC GGAGCCGTGCATACCAGAG GACTGGATTTCGCCTGCGA CATCTACATTTGGGCCCCTC TGGCTGGAACATGTGGCGT CCTGCTGCTGTCTCTCGTGA TCACCCTGTACTGC | Codon-optimized nucleotide sequence of the hinge and transmembrane domain amino acid sequence (138-206, NCBI accession # NP_001139345.1) of Human CD8α |
| 4 | CD28 nucleotide | AGAAGCAAGAGAAGCCGG CTGCTGCACAGCGACTACA TGAACATGACCCCTAGACG GCCCGGACCTACCAGAAAG CACTACCAGCCTTACGCTCC TCCTCGGGACTTTGCCGCCT ATAGATCC | Optimized nucleotide sequence of the intracellular signaling domain amino acid sequence (83-123, NCBI accession # NP_001230006.1) derived from CD28 |
| 5 | 4-1BB nucleotide | AAGCGGGGCAGAAAGAAG CTGCTGTACATCTTCAAGCA GCCCTTCATGCGGCCCGTG CAGACCACACAAGAGGAAG ATGGCTGCTCCTGCAGATTC CCCGAGGAAGAAGAAGGC GGCTGCGAGCTG | Optimized nucleotide sequence of the intracellular signaling domain amino acid sequence (214-255, NCBI accession # NP_001552.2) derived from 4-1BB |
| 6 | CD3ζ nucleotide | AGAGTGAAGTTCTCTAGAT CTGCCGACGCTCCTGCCTAC CAGCAGGGACAGAATCAGC TGTACAACGAGCTGAATCT GGGGCGCAGAGAAGAGTAC GACGTGCTGGATAAGAGAA GAGGCAGAGATCCCGAGAT GGGCGGCAAGCCCCAGAGA AGAAAGAATCCCCAAGAGG GCCTGTATAATGAGCTGCA GAAAGACAAGATGGCCGAG GCCTACAGCGAGATCGGAA TGAAGGGCGAACGCAGAAG AGGAAAGGGCCACGACGG ACTGTATCAGGGCCTGAGC ACAGCCACCAAGGATACCT ATGATGCCCTGCACATGCA GGCCCTGCCACCTAGG | Optimized nucleotide sequence of the intracellular signaling domain amino acid sequence (60-172, NCBI accession # XP_011508446.1) derived from CD3ζ |
| 45 | E1 monomer nucleotide | GTTTCTGATGTTCCGAGGG ACCTGGAAGTTGTTGCTGC GACCCCCACCAGCCTACTG ATCAGCTGGTATTATCCGTT TTGTGCGTTTTATTACAGGA TCACTTACGGAGAAACAGG AGGAAATAGCCCTGTCCAG GAGTTCACTGTGCCTCGTCC GTCTGACACTGCTACCATC AGCGGCCTTAAACCTGGAG TTGATTATACCATCACTGTG TATGCTGTCACGTGTCTGGG CTCGTATTCTCGCCCAATTT CCATTAATTACCGAACA | |

The nucleic acid sequences of said Table 1 may be expressed in an immune cell and expressed on the cell surface membrane as a monobody-based chimeric antigen receptor comprising the amino acid sequences of Table 2 below.

**[Table 2]**

| SEQ ID NO | Sequence Name | Sequence | Remarks |
|---|---|---|---|
| 7 | CD8α signal sequence amino acid | MALPVTALLLPLALLLHAAR P | Amino acid sequence corresponding to SEQ ID NO: 1 (amino acid sequence 1-21, NCBI accession # NP_741969.1) |
| 8 | hpE1 monomer amino acid | SSDSPRNLEVTNATPNSLTIS WYYPFCAFYYRITYGETGGN SPSQEFTVPRPSDTATISGLKP GQDYTITVYAVTCLGSYSRPI SINYRTEIDKPSQ | Amino acid sequence corresponding to SEQ ID NO: 2 |
| 9 | CD8α H&TM amino acid | TTTPAPRPPTPAPTIASQPLSL RPEACRPAAGGAVHTRGLDF ACDIYIWAPLAGTCGVLLLS LVITLYC | Amino acid sequence corresponding to SEQ ID NO: 3 (138-206, NCBI accession # NP_001139345.1) |
| 10 | CD28 amino acid | RSKRSRLLHSDYMNMTPRRP GPTRKHYQPYAPPRDFAAYR S | Amino acid sequence corresponding to SEQ ID NO: 4 (83-123, NCBI accession # NP_001230006.1) |
| 11 | 4-1BB amino acid | KRGRKKLLYIFKQPFMRPVQ TTQEEDGCSCRFPEEEEGGCE L | Amino acid sequence corresponding to SEQ ID NO: 5 (214-255, NCBI accession # NP_001552.2) |
| 12 | CD3ζ amino acid | RVKFSRSADAPAYQQGQNQ LYNELNLGRREEYDVLDKRR GRDPEMGGKPQRRKNPQEG LYNELQKDKMAEAYSEIGM KGERRRGKGHDGLYQGLST ATKDTYDALHMQALPPR | Amino acid sequence corresponding to SEQ ID NO: 6 (660-172, NCBI accession # XP_011508446.1) |
| 46 | E1 monomer amino acid | VSDVPRDLEVVAATPTSLLIS WYYPFCAFYYRITYGETGGN SPVQEFTVPRPSDTATISGLK PGVDYTITVYAVTCLGSYSR PISINYRT | Amino acid sequence corresponding to SEQ ID NO: 45 |

### (2) Preparation of Cells for Vector Cloning

Host cell for pMX-hpE1 vector: *E. coli* DH5α (F- *end*A1 *glnV44 thi-1 recA1 relA1 gyrA96 deoR nupG purB20* φ80d*lacZ*ΔM15 Δ (*lacZYA-argF)*U169, hsdR17(*rK-mK*+), λ-)
Host cell for pLentiviral vector: *E. coli Stbl3(F-mcrB mrrhsdS20(rB-,* mB-) *recA13 supE44* ara-14 *galK2 lac*Y1 *proA2 rps*L20(Str^{R}) *xyl-5* λ*leumtl*-1), Invitrogen^{™}

Said host cells were cultured at 37°C and 200 rpm using LB (Luria-Bertani) solid medium (Difco Laboratories, USA), and said host cells were prepared by adding 100 µg/ml of kanamycin or ampicillin to all media. Subsequently, a single colony of each of said cultured host cells was inoculated into LB liquid medium (Difco Laboratories, USA) for pre-culture, and this was inoculated at 1% into SOB (Super Optimal Broth) medium, cultured at 18°C for 24 hours, and then prepared by washing with Inoue TB buffer.

### (3) Preparation of Transformed Cells for Mass Production of Vectors

Said prepared vectors and said host cells were mixed, then allowed to stand on ice for 10 minutes, followed by treatment at 42°C for 50 seconds on ice for 3 minutes to prepare transformed cells into which each vector was introduced. Said prepared transformed cells were spread on said LB solid medium containing antibiotics and then cultured at 37°C. Through this process, each vector was prepared in large quantities.

### 1-2. Preparation of Recombinant Lentiviral Vectors Expressing Hydrophilic E1 Monobody-Chimeric Antigen Receptor (hpE1 CAR)

PCR was performed using said two prepared lentiviral vectors (pLenti7.3::CD8α leader sequence-E1 monobody-CD8 H&T-CD28-CD3ζ vector and pLenti7.3::CD8α leader sequence-E1 monobody-CD8 H&T-4-1BB-CD3( vector) as templates, along with the CPL-F primer and CPL-R primer shown in Table 3 below. Said amplified vectors were isolated and purified to prepare two types of scFv-free chimeric antigen receptor vectors.

The hpE1 monobody gene sequence was prepared by performing PCR on the pMX-hpE1 vector using the hpE1-F primer and hpE1-R primer shown in Table 3 below to amplify the sequence, followed by isolation and purification.

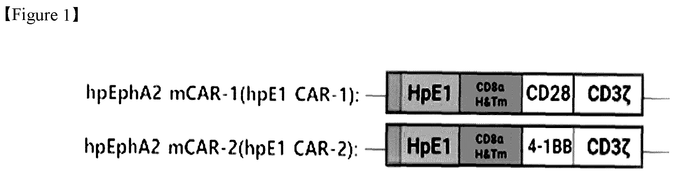

Two types of recombinant lentiviral vectors (pLenti7.3::CD8α leader-hpEphA2 monobody chimeric antigen receptor vectors) expressing chimeric antigen receptors containing the hpE1 monobody were prepared by inserting the hpE1 monobody sequence into the site where the E1 monobody sequence had existed, using the prepared vectors and hpE1 monobody sequence through a homologous recombination method with the Overlap cloner DNA cloning kit (Elpisbio). The prepared recombinant lentiviral vectors were named hpEphA2 mCAR-1 (hpVEC-1) and hpEphA2 mCAR-2 (hpVEC-2), respectively, and their structures are shown in FIG 1.

**[Table 3]**

| SEQ ID NO | Sequence Name | Sequence | Remarks |
|---|---|---|---|
| 13 | CPL F | ACCACCACACCAGCTCC | Linearization of plasmid and removal of ScFv sequence |
| 14 | CPL R | AGGTCTAGCAGCATGCAG | Linearization of plasmid and removal of ScFv sequence |
| 15 | hpE1 F | CATGCTGCTAGACCTAGCA GCGACAGCCCC | Amplification of hpE1 monobody |
| 16 | hpE1 R | AGCTGGTGTGGTGGTTTGG CTGGGCTTGTCGAT | Amplification of hpE1 monobody |

### [Example 2]

### Comparison between hpE1 Monobody and E1 Monobody

### 2-1. Sequence and Structural Alignment of hpE1 Monobody and E1 Monobody

Amino acid sequence alignment between the hpE1 monobody and E1 monobody was performed using Clustal Omega. The aligned sequences were processed through the Jalview program, and the identical sequences between the hpEl monobody and E1 monobody were indicated.

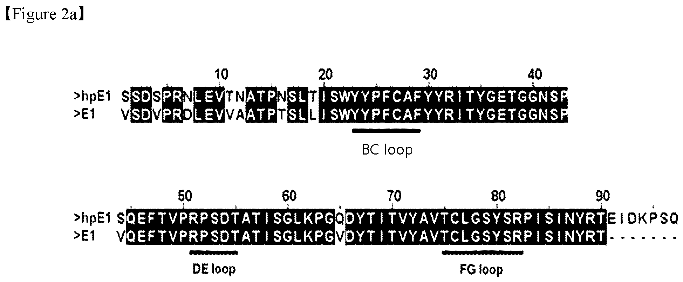

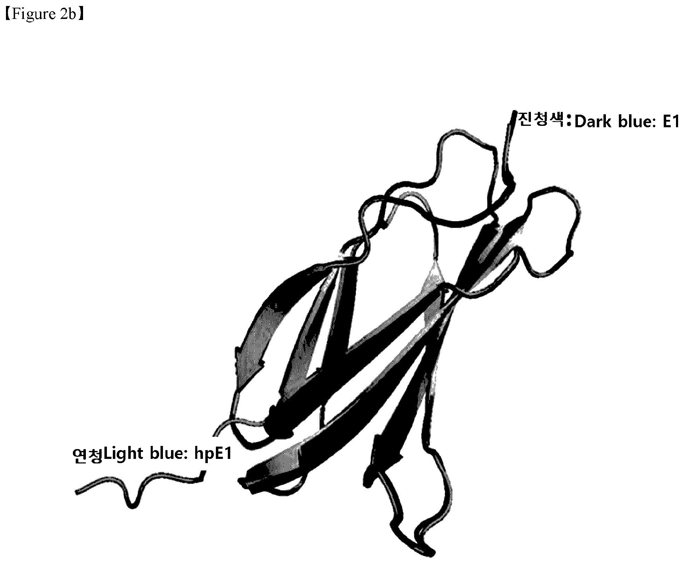

AlphaFold was used to obtain the predicted structures of the hpE1 monobody and E1 monobody. For the predicted structures, the relaxed structure corresponding to the top-ranked model was used. To evaluate the structural similarity between the hpE1 monobody and E1 monobody, the superimposition method of pymol was used. FIGS 2a and 2b show the protein sequence differences and overlaps between the hpE1 and E1 monobodies.

### 2-2. Comparison of Immunogenicity between hpE1 Monobody and E1 Monobody

Database analysis and IEDB MHC-II epitope binding prediction were performed for the monobody sequences binding to EphA2. As shown in Table 4 below, according to the IEDB MHC-II binding prediction method, overall immunogenicity of both hpE1 and E1 monobodies is predicted to be low; however, due to the hydrophobic amino acids introduced into the BC Loop, the E1 monobody is predicted to have a relatively higher potential for antigen presentation at the junction of the B beta-strand and the BC Loop compared to the hpE1 monobody.

**[Table 4]**

| Position | | hpE1 monobody peptide | Epitope Score | E1 monobody peptide | Epitope Score |
|---|---|---|---|---|---|
| | 1 | SSDSPRNLEVTNATP | 0 | VSDVPRDLEVVAATP | 1 |
| | 2 | SDSPRNLEVTNATPN | 0 | SDVPRDLEVVAATPT | 2 |
| | 3 | DSPRNLEVTNATPNS | 0 | DVPRDLEVVAATPTS | 2 |
| | 4 | SPRNLEVTNATPNSL | 2 | VPRDLEVVAATPTSL | 7 |
| | 5 | PRTLEVTNATPNSLT | 2 | PRDLEVVAATPTSLL | 7 |
| | 6 | RNLEVTNATPNSLTI | 3 | RDLEVVAATPTSLLI | 7 |
| | 7 | NLEVTNATPNSLTIS | 2 | DLEVVAATPTSLLIS | 5 |
| | 8 | LEVTNATPNSLTISW | 1 | LEVVAATPTSLLISW | 3 |
| | 9 | EVTNATPNSLTISWY | 1 | EVVAATPTSLLISWY | 0 |
| | 10 | VTNATPNSLTISWYY | 0 | VVAATPTSLLISWYY | 0 |
| | 11 | TNATPNSLTISWYYP | 0 | VAATPTSLLISWYYP | 0 |
| | 12 | NATPNSLTISWYYPF | 1 | AATPTSLLISWYYPF | 2 |
| | 13 | ATPNSLTISWYYPFC | 2 | ATPTSLLISWYYPFC | 5 |
| | 14 | TPNSLTISWYYPFCA | 4 | TPTSLLISWYYPFCA | 5 |
| | 15 | PNSLTISWYYPFCAF | 4 | PTSLLISWYYPFCAF | 5 |
| | 16 | NSLTISWYYPFCAFY | 4 | TSLLISWYYPFCAFY | 5 |
| | 17 | SLTISWYYPFCAFYY | 5 | SLLISWYYPFCAFYY | 6 |
| | 18 | LTISWYYPFCAFYYR | 5 | LLISWYYPFCAFYYR | 6 |
| | 19 | TISWYYPFCAFYYRI | 6 | LISWYYPFCAFYYRI | 6 |
| | 20 | ISWYYPFCAFYYRIT | 8 | ISWYYPFCAFYYRIT | 8 |
| | 21 | SWYYPFCAFYYRITY | 7 | SWYYPFCAFYYRITY | 7 |
| BC loop | 22 | WYYPFCAFYYRITYG | 4 | WYYPFCAFYYRITYG | 4 |
| | 23 | YYPFCAFYYRITYGE | 3 | YYPFCAFYYRITYGE | 3 |
| | 24 | YPFCAFYYRITYGET | 2 | YPFCAFYYRITYGET | 2 |
| | 25 | PFCAFYYRITYGETG | 1 | PFCAFYYRITYGETG | 1 |
| | 26 | FCAFYYRITYGETGG | 1 | FCAFYYRITYGETGG | 1 |
| | 27 | CAFYYRITYGETGGN | 0 | CAFYYRITYGETGGN | 0 |
| | 28 | AFYYRITYGETGGNS | 0 | AFYYRITYGETGGNS | 0 |
| | 29 | FYYRITYGETGGNSP | 0 | FYYRITYGETGGNSP | 0 |
| | 30 | YYRITYGETGGNSPS | 0 | YYRITYGETGGNSPV | 0 |
| | 31 | YRITYGETGGNSPSQ | 0 | YRITYGETGGNSPVQ | 0 |
| | 32 | RITYGETGGNSPSQE | 0 | RITYGETGGNSPVQE | 0 |
| | 33 | ITYGETGGNSPSQEF | 0 | ITYGETGGNSPVQEF | 1 |
| | 34 | TYGETGGNSPSQEFT | 0 | TYGETGGNSPVQEFT | 2 |
| | 35 | YGETGGNSPSQEFTV | 0 | YGETGGNSPVQEFTV | 2 |
| | 36 | GETGGNSPSQEFTVP | 0 | GETGGNSPVQEFTVP | 2 |
| | 37 | ETGGNSPSQEFTVPR | 0 | ETGGNSPVQEFTVPR | 0 |
| | 38 | TGGNSPSQEFTVPRP | 0 | TGGNSPVQEFTVPRP | 0 |
| | 39 | GGNSPSQEFTVPRPS | 0 | GGNSPVQEFTVPRPS | 0 |
| | 40 | GNSPSQEFTVPRPSD | 0 | GNSPVQEFTVPRPSD | 0 |
| | 41 | NSPSQEFTVPRPSDT | 0 | NSPVQEFTVPRPSDT | 0 |
| | 42 | SPSQEFTVPRPSDTA | 0 | SPVQEFTVPRPSDTA | 0 |
| | 43 | PSQEFTVPRPSDTAT | 0 | PVQEFTVPRPSDTAT | 0 |
| | 44 | SQEFTVPRPSDTATI | 0 | VQEFTVPRPSDTATI | 0 |
| | 45 | QEFTVPRPSDTATIS | 0 | QEFTVPRPSDTATIS | 0 |
| | 46 | EFTVPRPSDTATISG | 0 | EFTVPRPSDTATISG | 0 |
| | 47 | FTVPRPSDTATISGL | 0 | FTVPRPSDTATISGL | 0 |
| | 48 | TVPRPSDTATISGLK | 0 | TVPRPSDTATISGLK | 0 |
| | 49 | VPRPSDTATISGLKP | 0 | VPRPSDTATISGLKP | 0 |
| | 50 | PRPSDTATISGLKPG | 0 | PRPSDTATISGLKPG | 0 |
| DE loop | 51 | RPSDTATISGLKPGQ | 0 | RPSDTATISGLKPGV | 0 |
| | 52 | PSDTATISGLKPGQD | 0 | PSDTATISGLKPGVD | 0 |
| | 53 | SDTATISGLKPGQDY | 0 | SDTATISGLKPGVDY | 0 |
| | 54 | DTATISGLKPGQDYT | 0 | DTATISGLKPGVDYT | 0 |
| | 55 | TATISGLKPGQDYTI | 0 | TATISGLKPGVDYTI | 1 |
| | 56 | ATISGLKPGQDYTIT | 0 | ATISGLKPGVDYTIT | 1 |
| | 57 | TISGLKPGQDYTITV | 0 | TISGLKPGVDYTITV | 1 |
| | 58 | ISGLKPGQDYTITVY | 0 | ISGLKPGVDYTITVY | 0 |
| | 59 | SGLKPGQDYTITVYA | 0 | SGLKPGVDYTITVYA | 0 |
| | 60 | GLKPGQDYTITVYAV | 0 | GLKPGVDYTITVYAV | 1 |
| | 61 | LKPGQDYTITVYAVT | 1 | LKPGVDYTITVYAVT | 2 |
| | 62 | KPGQDYTITVYAVTC | 2 | KPGVDYTITVYAVTC | 2 |
| | 63 | PGQDYTITVYAVTCL | 3 | PGVDYTITVYAVTCL | 2 |
| | 64 | GQDYTITVYAVTCLG | 2 | GVDYTITVYAVTCLG | 2 |
| | 65 | QDYTITVYAVTCLGS | 1 | VDYTITVYAVTCLGS | 0 |
| | 66 | DYTITVYAVTCLGSY | 0 | DYTITVYAVTCLGSY | 0 |
| | 67 | YTITVYAVTCLGSYS | 0 | YTITVYAVTCLGSYS | 0 |
| | 68 | TITVYAVTCLGSYSR | 0 | TITVYAVTCLGSYSR | 0 |
| | 69 | ITVYAVTCLGSYSRP | 0 | ITVYAVTCLGSYSRP | 0 |
| | 70 | TVYAVTCLGSYSRPI | 0 | TVYAVTCLGSYSRPI | 0 |
| | 71 | VYAVTCLGSYSRPIS | 0 | VYAVTCLGSYSRPIS | 0 |
| | 72 | YAVTCLGSYSRPISI | 1 | YAVTCLGSYSRPISI | 1 |
| | 73 | AVTCLGSYSRPISIN | 1 | AVTCLGSYSRPISIN | 1 |
| | 74 | VTCLGSYSRPISINY | 1 | VTCLGSYSRPISINY | 1 |
| | 75 | TCLGSYSRPISINYR | 1 | TCLGSYSRPISINYR | 1 |
| | 76 | CLGSYSRPISINYRT | 1 | CLGSYSRPISINYRT | 1 |

### 2-3. Measurement of Binding Affinity of hpE1 Monobody to EphA2 Antigen Protein

### 1) Purification of hydrophilic E1 monobody and E1 monobody proteins in E. coli

To construct E. coli expression vectors for monobody proteins, hydrophilic E1 monobody and E1 monobody genes were amplified by PCR. The amplified PCR fragments were digested at both ends with NdeI and BamHI restriction enzymes and ligated into the restriction enzyme sites of the pETamh (AviTag-Myc-6xHis) expression vector. The pETamh expression vector derived from pET (Novagen, USA) has an NdeI restriction enzyme site including the ATG start codon located at the 5' end, and at the 3' end, a site corresponding to the amh tag is located after the BamHI restriction enzyme site and before the stop codon. Accordingly, the monobodies expressed with the amh tag linked to the C-terminus of the monobody are named hpE1-amh and E1-amh, respectively. The hpE1 monobody and E1 monobody inserted into the pETamh expression vector were transformed into BL21(DE3) and expressed. A single colony was inoculated into 10 ml of LB medium containing kanamycin (50 µg/ml), cultured overnight, and harvested through centrifugation. After resuspending in 1 mL of LB medium and 1 mL of glycerol (50%), the strain dispensed in 200 µL aliquots was inoculated into 1 L of LB medium and cultured at 37°C and 250 rpm until the OD value reached between 0.5 and 0.7. After adding 1 ml of 0.5M IPTG to the cultured E. coli culture medium, additional cultivation was performed overnight at 20°C and 250 rpm. The cultured E. coli was centrifuged at 4°C and 8,000 rpm for 10 minutes to obtain the E. coli precipitate. 10 ml of cold lysis buffer (20 mM Tris-HCl (7.4), 100 mM NaCl) was added per 1 g of the E. coli precipitate to lyse the E. coli precipitate, followed by centrifugation at 13,000 rpm for 30 minutes.
The hpE1-amh and E1-amh in the obtained supernatant were separated through imidazole gradient elution on a Biorad FPLC equipped with a HisTrap column (EconoFit Nuvia IMAC Columns, Ni-charged 5 ml). The primary purified monobody proteins were further separated in PBS buffer using size-exclusion chromatography (Superdex^{™} 75 Increase 10/300 GL).

### [Example 3]

### Preparation of Immune Cells Expressing hpE1 Monobody-Chimeric Antigen Receptor on the Cell Membrane Surface (hpVEC-1 and VEC-1) and Comparative Evaluation of Transduction Efficiency

### 3-1. Preparation of Lentivirus Containing Recombinant Lentiviral Vectors

Lentiviruses containing hpEphA2 mCAR-1 and EphA2 mCAR-1 prepared in Example 1 were prepared. Lentivirus production and Titration were performed using the LV-MAX^{™} Lentiviral Production System (A35684) kit provided by Thermo Scientific and were prepared according to the manufacturer's experimental methods.

### 3-2. Preparation of Immune Cells and Transduction Through Viral Infection

### (1) Isolation of CD3+ T cells from PBMC

Peripheral blood mononuclear cells (PBMC) were isolated from human donor blood using Lymphoprep^{™} (STEMCELL). Subsequently, human CD3+ T cells were isolated from the isolated PBMC by a positive selection method using CD3 microbeads (Miltenyi Biotech).

### (2) Activation of CD3+ T cells

Anti-CD3/anti-CD28 magnetic beads (anti-CD3/CD28 Dynabead; Gibco) and the isolated human CD3+ T cells were mixed at a 1:1 ratio. After 24 hours of mixing, the activation magnetic beads were removed using a MACSiMAG separator device. Subsequently, the cells were washed using RPMI-1640.

### (3) Transduction with lentivirus

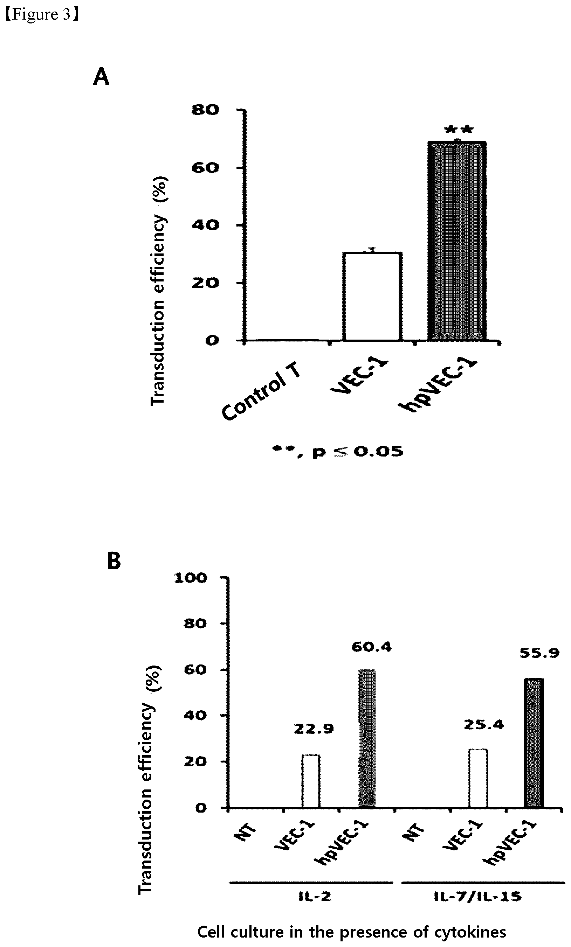

T cells infected with lentivirus were prepared by a viral infection method using the spinoculation technique with a centrifuge. Specifically, activated human CD3+ T cells were infected with lentiviruses containing hpEphA2 mCAR-1 and EphA2 mCAR-1, respectively, and the virus medium was replaced after 24 hours. Cell proliferation begins 48 hours after infection. The engineered immune cells prepared in this manner were named hpVEC-1 and VEC-1, respectively. An Attune NxT flow cytometer (Thermo Fisher Scientific) was used to test the expression rate of the transduced vector (i.e., the hpE1 monobody-chimeric antigen receptor sequence). As shown in FIG. 3, as a result of comparative analysis of the number of GFP-expressing cells by FACS, it was confirmed that in the culture condition supplemented with IL-2, hpVEC-1 immune cells showed an expression rate of approximately 60%, and E1 monobody-chimeric antigen receptor VEC-1 immune cells showed an expression rate of approximately 23%, compared to the control group in which no vector was introduced. In addition, in the culture condition supplemented with IL-7 and IL-15, hpVEC-1 showed an expression rate of approximately 56%, and VEC-1 showed an expression rate of approximately 25%, compared to the control group in which no vector was introduced. From these results, it can be confirmed that the vector introduced into the immune cells is well expressed, meaning that the number of cells expressing the hpE1 monobody is more than twice as high as that of the E1 monobody. These results mean that in the treatment of patients having cells expressing the cancer antigen EphA2, hpVEC-1 and hpVEC-2 are more likely to recognize EphA2-expressing cancer cells much faster or in greater numbers than VEC-1 and VEC-2.

### [Example 4]

### Comparative Evaluation of Cell Activity of hpVEC-1 and VEC-1

Whether the hpEphA2 mCAR-1 and EphA2 mCAR-1 vectors introduced in the above-prepared hpVEC-1 and VEC-1 immune cells were properly expressed, and the activation status of these immune cells, were confirmed using an Attune NxT flow cytometer (Fluorescence-activated cell sorting).

For each immune cell, the expression of the introduced vector was confirmed using an anti-GFP (Green Fluorescent Protein) antibody. Since a GFP expression site is present in the corresponding vector, immune cells that highly express such a vector will show high measurement results in the analysis using the anti-GFP antibody. Additionally, the activation of the T cells was confirmed using an anti-CD62L-APC antibody (APC Mouse Anti-Human CD62L, BD Pharmingen^{™}), an anti-CD25-PE antibody (PE Mouse Anti-Human CD25, BD Pharmingen^{™}), and an anti-CD69-APC antibody (APC Mouse Anti-Human CD69, BD Pharmingen^{™}).

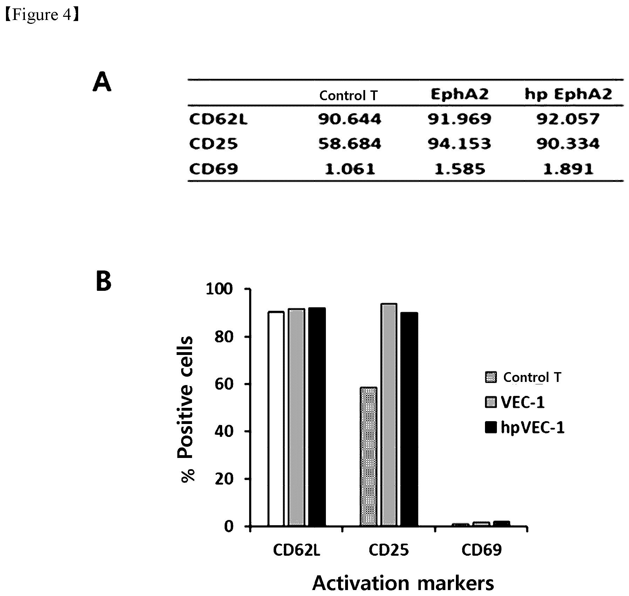

As shown in FIG. 4, in the case of the in vitro test, it was confirmed that in the engineered immune cells hpVEC-1 and VEC-1, CD62L, a cell growth activity marker, was highly expressed at 85% or more, similar to the control group, and CD25 was expressed 20% or higher than the control group.

### 2) Measurement of Binding Affinity (Kd Dissociation Constant) of Purified Monobody Proteins

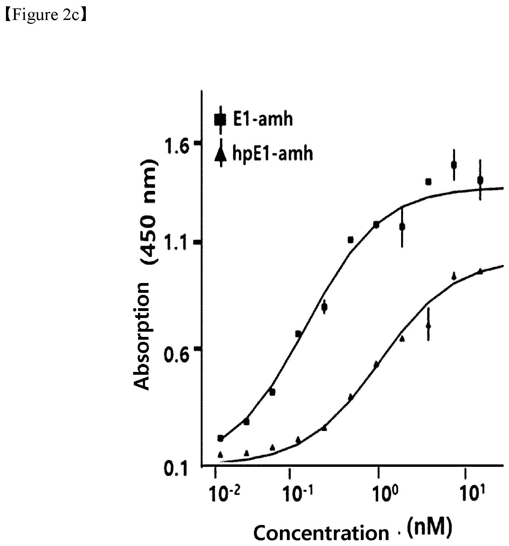

The binding affinity of the hpE1 monobody to hEphA2 was evaluated by measuring the dissociation constant (Kd) through ELISA analysis. 1 µg/mL of recombinant EphA2 protein (R&D systems) was dispensed into each well of a 96-well ELISA plate (Costar) and coated overnight at 4°C. After coating, each well was washed three times with PBST containing 0.05% Tween 20 and treated with 200 µL of blocking buffer (PBST containing 5% BSA) at room temperature for 2 hours. Each well was washed three times with PBST, and hpE1-amh and E1-amh monobody proteins were added by serial dilution by half the concentration starting from 500 nM and allowed to bind for 2 hours at room temperature. Each well was washed with PBST and then treated with 100 ul of HRP-conjugated c-Myc monoclonal antibody (Invitrogen) at room temperature for 1 hour. After washing, each well was reacted with 100 ul of substrate [a 1:1 mixture of substrate (TMB) and hydrogen peroxide, BD Biosciences] at room temperature for 5 minutes. The reaction was stopped with 50 uL of 1 M H₂SO₄, and the degree of color development was read at a wavelength of 450 nm with an ELISA reader. As shown in FIG. 2c, it was confirmed that the binding affinity of hpE1 to hEphA2 was stronger than that of E1.

### [Example 5]

### Comparative Evaluation of the Expression of Cell Inhibitory Factors of hpVEC-1 and VEC-1

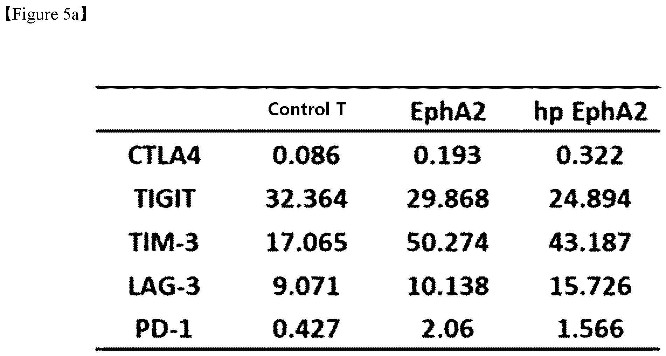

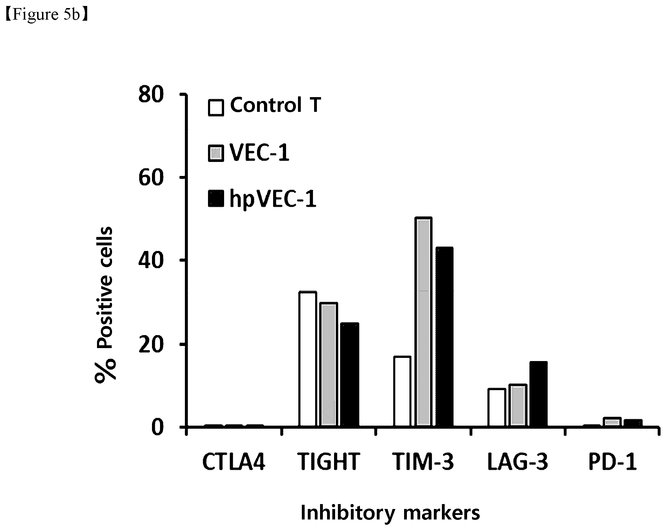

To compare the expression of inhibitory factors in T cells expressing hydrophilic VEC-1 and conventional VEC-1, anti-CTLA-4-APC antibody, anti-TIGIT-APC antibody, anti-TIM-1-BV421 antibody, anti-LAG-1-PE antibody, and anti-PD-1-PE antibody were diluted 1:100 using FACS buffer, reacted with 1 x 10⁶ of each CAR-T cell at 4°C for 30 minutes, washed twice with 200 ul of FACS buffer, and then the expression of each protein was analyzed by FACS. As shown in FIGs. 5a and 5b, the expression of CTLA4 or PD-1 was very low in all immune cells expressing conventional VEC-1 or hydrophilic VEC-1, including the control group in which the virus was not introduced; TIGIT and TIM-3 were expressed approximately 5~10% lower in immune cells expressing hydrophilic VEC-1 compared to conventional VEC-1, respectively, while in the case of LAG-3, there was a difference of about 5.73% compared to cells expressing conventional VEC-1.

### [Example 6]

### Evaluation of In Vitro Cytotoxicity of hpVEC-1 and VEC-1 in EphA2-Overexpressing HEK293 Cell Lines

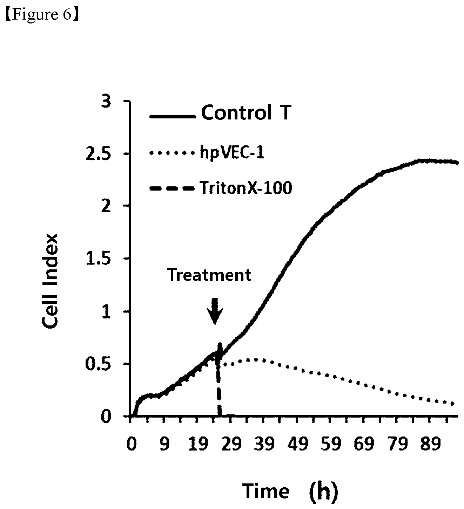

To confirm the killing ability of T cells expressing hydrophilic VEC-1 against cells expressing EphA2, an EphA2-overexpressing HEK293 stable cell line was generated. A plasmid in which EphA2 cDNA was cloned was purchased from addgen (#116732) and introduced into HEK293 cells using lipofectamine 2000. After 48 hours, cells in which the plasmid was integrated into the HEK293 cell genome were selected by treatment with 800ug/ml of G418, and then single cells were transferred one by one into a 96-well plate and cultured. Each clone proliferated from a single cell was confirmed to express the EphA2 protein in HEK293 cells through FACS analysis using an EphA2-PE antibody. To measure the cytotoxicity of each VEC-1-expressing T cell against the EphA2-overexpressing HEK293 cells, real-time cell analysis (RTCA) was performed. 1 x 10⁴ HEK293 cells per 100 ul were cultured in a microtiter plate and monitored for 24 hours. After 24 hours, an equal number of hydrophilic VEC-1-expressing T cells were treated, and the killing ability of the hydrophilic VEC-1-expressing T cells was confirmed through RTCA. As shown in FIG. 6, T cells that do not express CAR (control group) had no effect, but the hydrophilic VEC-1-expressing T cells showed an effect of killing almost all of the EphA2-expressing HEK293 cells.

### [Example 7]

### Evaluation of In Vitro Cytotoxicity Against Cancer Cells

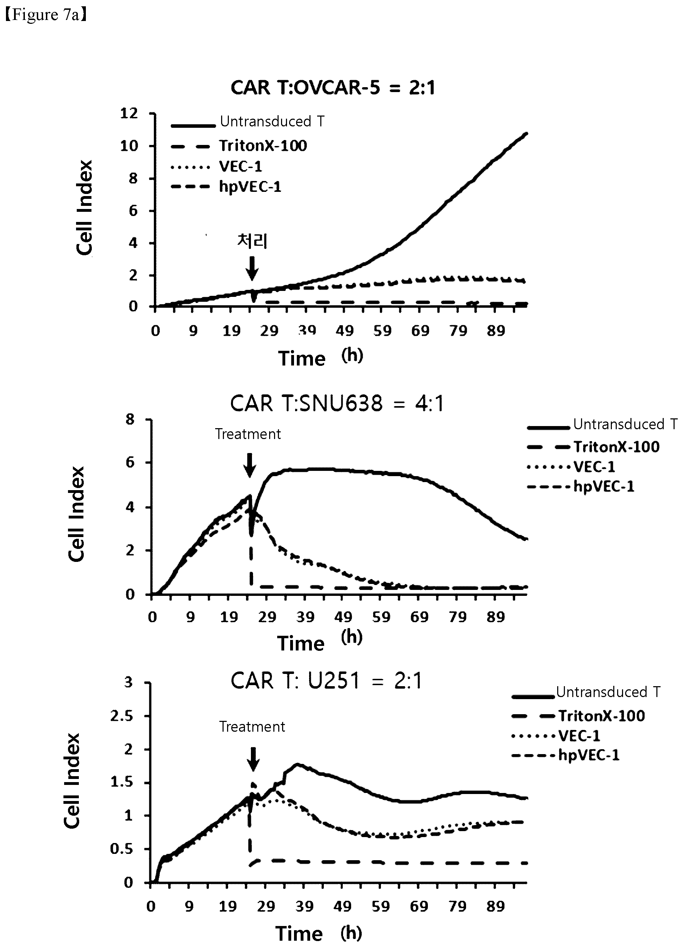

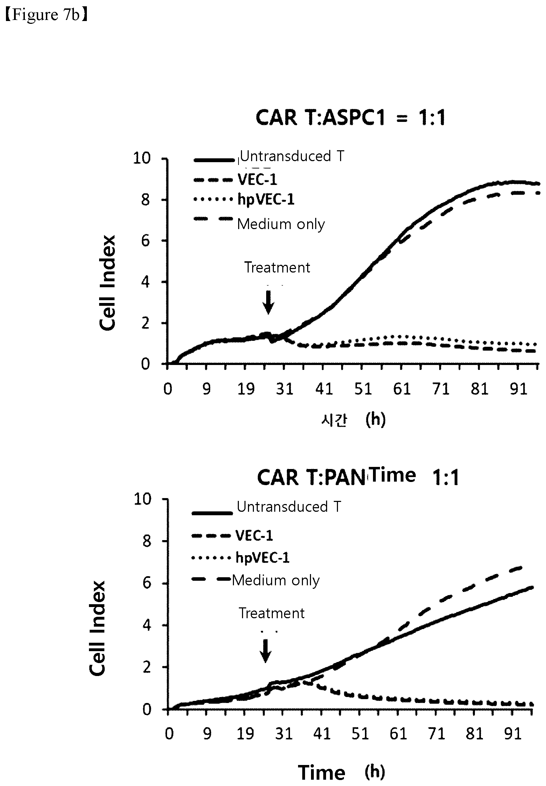

To compare cytotoxicity, each CAR-T cell was treated on ovarian cancer cell line (OVCAR-5), gastric cancer cell line (SNU638), glioblastoma cell line (U251), and pancreatic cancer cell lines (ASPC-1, Panc-1) purchased from the Korean Cell Line Bank, and then RTCA was performed in the same manner as in Example 6. As shown in FIGs. 7a and 7b, T cells expressing hydrophilic VEC-1 showed toxicity against all cancer cell lines, and an efficacy almost identical to that of T cells expressing conventional VEC-1 was confirmed.

### [Example 8]

### Evaluation of Stability for Cell Efficacy of hpVEC-1 and VEC-1 (Persistency)

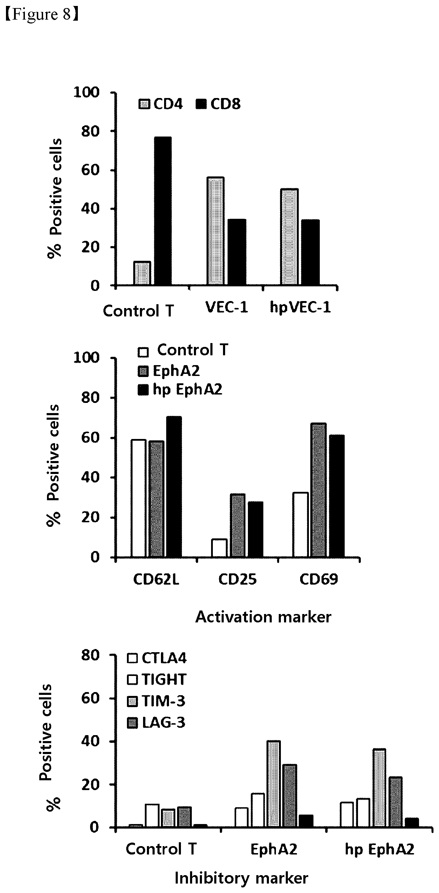

To investigate the persistency of each marker factor in T cells expressing conventional VEC-1 and T cells expressing hydrophilic VEC-1, T cells expressing each chimeric antigen receptor were engineered and cultured for 25 days, and then the ratio of CD4+: CD8+ cells and the markers for activation and inhibitory factors were compared using the antibodies used in said Examples 3 to 4. As shown in FIG. 8, compared to T cells into which CAR was not introduced (control group), the ratio of CD4+ showed a relatively higher value than the control group after 25 days, and there was no significant difference compared to conventional VEC-1. Regarding activation factor markers, CD62L was higher in T cells expressing hydrophilic VEC-1 than in T cells expressing conventional VEC-1, while CD25 and CD69 were slightly lower. Regarding the inhibitory factors, TIGIT, Tim3, and LAG-3 were expressed at lower levels in T cells expressing hydrophilic VEC-1 compared to T cells expressing conventional VEC-1.

### [Example 9]

### Evaluation of In Vitro Cytotoxicity Against Cancer Cells 24 Days After Production of hpVEC-1 and VEC-1

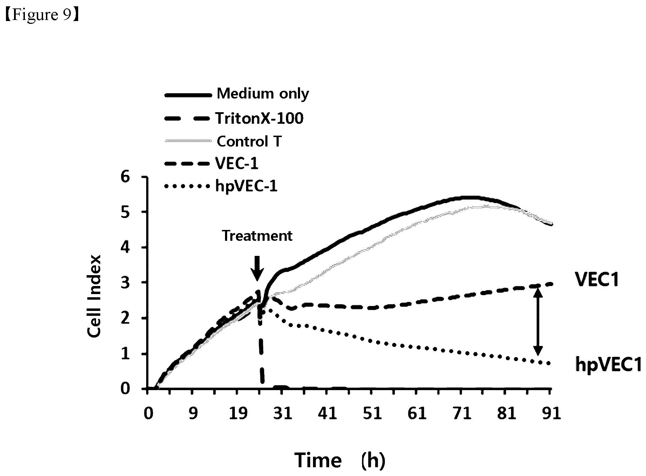

As a result of analyzing the cytotoxicity against gastric cancer cells of transduced hpE1 monobody CAR-T cells (hpVEC-1 or hpVEC-2), E1 monobody CAR-T cells (VEC-1 or VEC-2), and non-transduced human T cells (control group) by RTCA, as confirmed in Example 7, the cytotoxicity of hpVEC-1 (or hpVEC-2) produced after 9 days of production period was significantly higher than that of the control group, but was almost similar to that of VEC-1 (or VEC-2). However, after an additional culture period of 24 days from production, each was co-cultured with cancer cells using RTCA equipment, and the cytotoxicity was re-investigated. As shown in FIG. 9, hpVEC-1 (or hpVEC-2) with a 24-day culture period showed high cytotoxicity compared to the control group, and it was also confirmed that it maintained higher cytotoxicity compared to VEC-1 (or VEC-2). From these results, it was confirmed that the immune cells engineered with the hpE1 monobody according to the present invention stably maintain their activity for a long period of time.

### [Example 10]

### Comparison of Cytotoxicity of hpVEC-1 and VEC-1 Against Ovarian Cancer and Gastric Cancer Cells Through 3D Co-culture Method

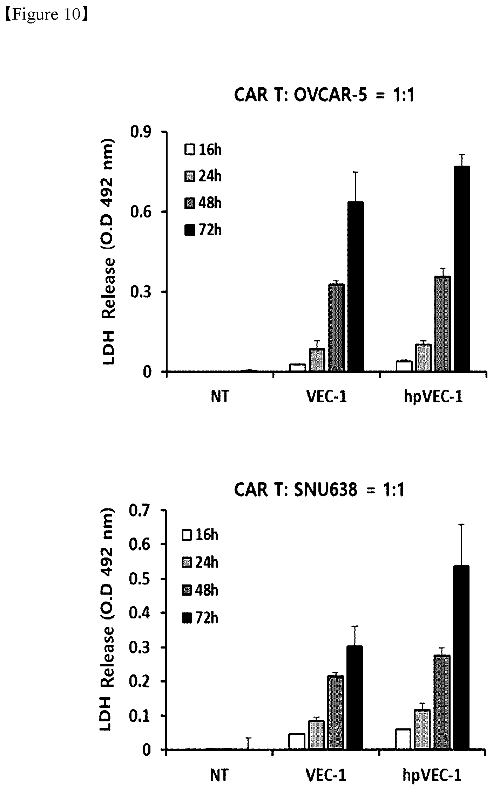

Since real-time cell analysis has limitations as an assay through two-dimensional culture, the functions of the two VEC-1 chimeric antigen receptor T cells were compared through a three-dimensional spheroid culture method that more closely mimics tumors. After mixing 0.5x10⁴ cells of each gastric cancer cell line and ovarian cancer cell line with RPMI medium containing 2.5% matrigel(Corning), they were placed in a round-bottom 96-well culture plate without cell adhesion ability, centrifuged at a speed of 1000 x g for 10 minutes, and then cultured for 48 hours. Since matrigel has a property of solidifying at room temperature, all processes were performed on ice. After confirming that spherical spheroids were formed by the reaction between matrigel and cells, each T cell expressing VEC-1 was treated, and after 16, 24, 48, and 72 hours, the medium was collected, and the amount of LDH contained in the medium was measured (promega). As shown in FIG. 10, in spheroids using the ovarian cancer cell line (OVCAR5), the amount of LDH gradually increased over time, but almost no difference was observed between the two cells; however, in spheroids using the gastric cancer cell line (SNU638), a higher amount of LDH was measured in the group treated with T cells expressing hydrophilic VEC-1 than in the group treated with conventional VEC-1. This shows that the function of T cells expressing hydrophilic VEC-1 provides improved cytotoxicity compared to T cells expressing conventional VEC-1.

### [Example 11]

### Evaluation of Anti-tumor Efficacy of Immune Cells in Xenograft Mouse Model

All animal experiments were conducted with the approval of the Institutional Animal Care and Use Committee (IACUC, Chonnam National University, Republic of Korea). Four-week-old female NOG mice were supplied from Koatech SPF animal laboratory.

The ovarian cancer cell OVCAR-5 was suspended at a concentration of 2 x 10⁵ cells/100ul in a solution mixed 1:1 with PBS(Welgene) and high-concentration matrigel(Corning). In the experiment, OVCAR-3 (2 x 10⁵ cells) was subcutaneously injected into the right flank of the mouse.

When the tumor size reached 50 ~ 100 mm³, the prepared PBS, control T cells, and VEC-1 and hpVEC-1 immune cells at a concentration of 5 x 10⁶/200ul were intravenously injected once. 4~7 mice were used per group, and the tumor size and body weight of the mice were measured twice a week.

### 11-1. Cell thawing and Cell culture

**Materials:** RPMI 1640(Gibco), FBS(Gibco), P/S(Gibco), L-glutamine 200mM(Gibco), 100∮ cell culture flask, basket, tumor cell line(OVCAR-5), Solution 18 AODAPI(ChemoMetec), PBS(Welgene), TrypLE^{™}(Gibco). The human ovarian cell line was supplied from the Korean Cell Line Bank.

**Cell thawing:** the tumor cell line was taken from a liquid nitrogen tank, placed in a 37°C water bath and thawed rapidly, and when thin ice was floating, it was moved to a clean bench and placed in a 15 mL conical tube. 9 mL of cell culture medium (RPMI 1640 + 10% FBS + 1% P/S + 2mM L-glutamine) was added to the 15 mL tube and centrifuged at 350g for 3 minutes. After removing the supernatant, 1 mL of cell culture medium was added for complete resuspension, and then 9 mL of cell culture medium was added to reach a final volume of 10 mL for resuspension. The number of cells was calculated using a cell counting solution (PBS : solution 18 = 90 : 5), and the cells were seeded in a 100∮ flask at cell densities adjusted to 0.5 x 10⁶/10 mL and 1 x 10⁶ /10 mL. Thereafter, subculturing was performed at 3-day intervals (when the cell density reached 70~80%).

**Subculturing:** after removing the supernatant from the flask, 10 mL of PBS was added to wash away impurities such as cell debris, and then the supernatant was discarded. To detach the cells from the flask, TrypLE^{™}(2 mL or 4 mL) was added to 100∮ and 150∮ flasks, respectively. When the cells were observed under a microscope to have a round shape and be floating, 10 mL or 30 mL of cell culture medium (RPMI 1640 + 10% FBS + 1% P/S + 2mM L-glutamine) was added, and the cells were collected in a 50 mL conical tube and centrifuged at 500g for 3 to 5 minutes. After removing the supernatant, 1 mL of cell culture medium was added for resuspension, and then 9 mL of cell culture medium was added. After measuring the number of cells using a cell counting solution (PBS : solution 18 = 90 : 5), cancer cells (for example, ovarian cancer cell OVCAR-5: 1x10⁶/10 mL) were placed in a culture vessel and cultured.

### 11-2. Mouse preparation

**Materials:** PICO 5053(Orient Bio), γ-irradiated 18% beta-chip(Orient Bio), sterilized tap water and water bottle, autoclave, clipper(Jeungdo B&P), wet wipes, ifran solution (Hana Pharm), hair removal cream (veet), inhalation anesthesia machine and chamber

**Experimental method:** feed (PICO 5053), bedding (r-irradiated 18% beta-chip), sterilized tap water, and cages were replaced twice a week. Hair was removed 3 days before tumor implantation, and during the experiment, it was removed when it grew enough to obscure the tumor. Mice were anesthetized by inhalation with an isoflurane concentration of 2~3 %, and the hair on the right flank of the mice was removed with a clipper, followed by applying hair removal cream, leaving it for about 1 minute, and wiping the hair removal cream off with wet wipes.

### 11-3. Preparation of human tumor xenograft mouse model

**Materials:** cells, 1 ml syringe (KOREAVACCINE), 27 ½ G needle (KOREAVACCINE), high-concentration matrigel(Corning), ice box, EP tube, inhalation anesthesia machine and chamber, ifran solution(Hana Pharm). In the case of matrigel, it was left overnight in a refrigerator one day before xenografting. Since matrigel gelatinizes at room temperature, the experiment was always conducted by placing it on ice.

**Experimental method:** 100 ul of a solution in which OVCAR-5(2 x 10⁵ cells) and matrigel were mixed 1:1 was subcutaneously injected into the right flank of mice anesthetized by inhalation with 2~3% isoflurane.

### 11-4. Injection of chimeric antigen receptor T cells

**Materials:** 27 ½ G needle(Jungrim), 1 ml syringe (KOREAVACCINE), PBS, normal T cells (control group), E1 monobody CAR-T cells(VEC-1), hydrophilic E1 monobody CAR-T cells(hpVEC-1), mouse restrainer(Jeungdo B&P), heat lamp (Jeungdo B&P), 70% alcohol swab, vernier caliper(Jeungdo B&P), scale, camera.

**Experimental method:** the tumor size and body weight of the mice were measured to balance the groups with similar tumor sizes and body weights. When the tumor size reached 50 ~ 100 mm³, 200 ul of PBS and cells (5 x 10⁶/200ul of control normal T cells, VEC-1, and hpVEC-1) were intravenously injected into the tail vein of the mice.

### 11-5. Mouse monitoring (tumor size and body weight measurement)

**Materials:** inhalation anesthesia machine and chamber, ifran solution (Hana Pharm), vernier caliper (Jeungdo B&P), scale, camera.

**Experimental method:** after anesthetizing mice by inhalation with 2~3% isoflurane, tumor size and body weight were measured twice a week. The size of the mouse tumor was calculated by measuring the long axis (L), short axis (W), and height (H) with a vernier caliper and multiplying by 0.52, and the body weight of the mice was measured using an electronic scale.

### 11-6. Confirmation of Cytotoxicity against ovarian cancer cell line

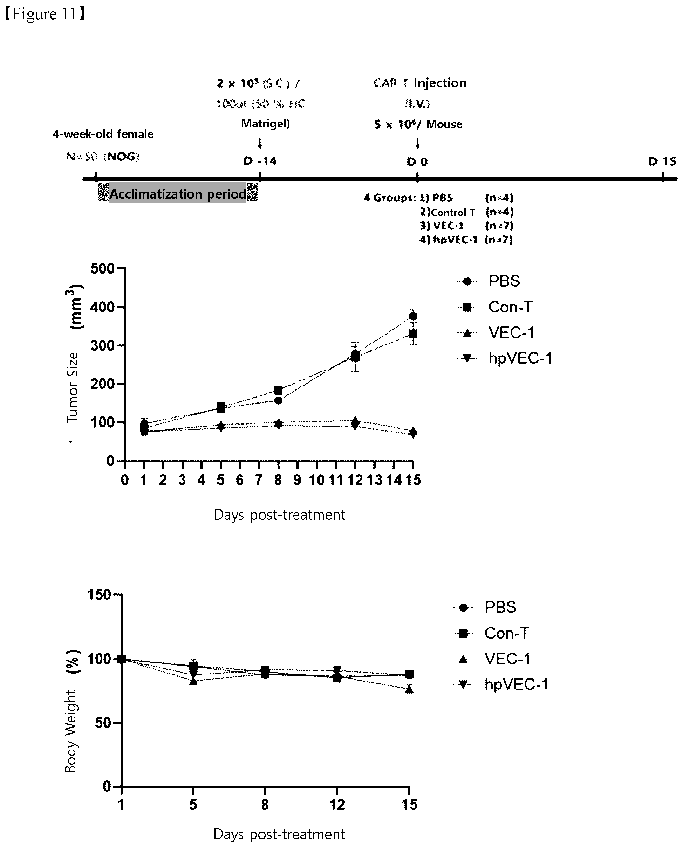

In mice model xenografting the ovarian cancer cell line (OVCAR-5) at 2 x 10⁵ cells/100ul into the subcutaneous tissue of the right back of the mice, 14 days after tumor generation, PBS, control T cells(activated control normal T cells not transduced with virus; Cont-T), VEC-1 cells, and hpVEC-1 cells were intravenously injected for each group. The tumor size was measured using a scale bar, and the results are shown in FIG. 11.

As shown in FIG. 11, in mice injected with VEC-1 or hpVEC-1, the tumor size significantly decreased starting from 14 days after tumor generation compared to mice injected with only control normal T cells or PBS.

In order to determine whether the mice's body condition recovered after intravenous administration of E1 monobody CAR-T cells and whether there were side effects due to the administration, the body weight of the mice was measured, and the results are shown in FIG. 11.

As shown in FIG. 11, the health of the mice administered with hpVEC-1 recovered more rapidly than that of the mice administered with control normal T cells or VEC-1 starting from 8 days after administration.

### [Example 12]

### Preparation of PD-L1 Single-Chain Fragment Antibody(VYPC)-Hydrophilic E1 Monobody Dual-Targeting Vector

### 12-1. Preparation for the production of recombinant lentiviral vectors

The following strains and vectors were prepared for the production of a recombinant plasmid vector expressing a hydrophilic monobody-based chimeric antigen receptor.

A vector expressing the chimeric antigen receptor was prepared by synthesizing and using a pLenti7.3-VYPC vector containing the PD-L1 single-chain fragment antibody(VYPC) nucleic acid sequence(Y-Biologics) of SEQ ID NO: 18, which specifically binds to human PD-L1, and a pMX-hpE1 vector containing the hpE1 monobody nucleic acid sequence(SEQ ID NO: 20), which specifically binds to human EphA2, from Thermo Fisher (GeneArt). Specifically, the pLenti7.3/V5-DEST vector from Invitrogen was used as the expression vector, and it was designed to include a nucleic acid sequence expressing a chimeric antigen receptor consisting of CD8α leader sequence-E1 monobody sequence-CD8 H&TM-CD28-CD3ζ or CD8α leader sequence-E1 monobody sequence-CD8 H&TM-4-1BB-CD3ζ between the 5' UTR and 3' UTR of the pLenti7.3/V5-DEST vector. The nucleic acid sequences constituting the monobody-based chimeric antigen receptor in the recombinant plasmid vector are shown in table 5 below.

**[Table 5]**

| SEQ ID NO | Sequence Name | Sequence | Remarks |
|---|---|---|---|
| 17 | CD8 *α* signal sequence nucleotide | ATGGCCCTGCCTGTGAC AGCTTTGCTGCTGCCTC TGGCTCTGCTGCTGCAT GCTGCTAGACCT | Codon-optimized nucleotide sequence of the signal sequence domain amino acid sequence (1-21, NCBI accession # NP_741969.1) of Human CD8α |
| 18 | PD-L1 scFv(VYPC) nucleotide | CAGATGCAGCTGGTGCA GTCTGGGGCTGAGGTGA AGAAGCCTGGGTCCTCG GTGAAGGTCTCCTGCAA GGCTTCTGGAGGCACCT TCAGCAGCTATGCTATC AGCTGGGTGCGACAGGC CCCTGGACAAGGGCTTG AGTGGATGGGAAGGATC ATCCCTATCCTTGGTAT AGCAAACTACGCACAGA AGTTCCAGGGCAGAGTC ACGATTACCGCGGACAA ATCCACGAGCACAGCCT ACATGGAGCTGAGCAGC CTGAGATCTGAGGACAC GGCCGTGTATTACTGTG CGAAACCGAGAGATGGC TACAATTTGGTTGCTTT TGATATCTGGGGCCAAG GGACGATGGTCACCGTC TCCTCAGGCCTCGGGGC CTCGGAGGAGGAGGTAG TGGCGGAGGAGGCTCCG GTGGATCCAGCGGTGTG GGTTCCCAGCTCGTGCT GACTCAGCCGCCCTCAGT GTCTGGGGCCCCAGGGCA GAGGGTCACCATCTCCT GCACTGGGAGCAGCTCC AACATCGGGGCAGGTTA TGATGTACACTGGTATC AGCAACTTCCAGGAGCA GCCCCCAAACTCCTCATC TATGGCGACATCAATCG GCCCTCAGGGGTCCCTGA CCGATTCTCTGGCTCCA AGTCTGGCATCTCAGCC TCCCTGGCTATCACTGG GCTCCAGGCTGAGGACG AGGCTGATTATTACTGC CAGTCCTATGACAGCAG CCTGAGTGGGGGGTGT TCGGCGGAGGGACCAAG CTGACCGTCCTA | |
| 19 | Linker | GGAGGCGGAGGTTCTGG CGGCGGAGGAAGTGGTG GCGGAGGCTCT | Ligation of VYPC and hpE1 monomer nucleotides |
| 20 | hpE1 monomer nucleotide | AGCAGCGACAGCCCCAGA AATCTGGAAGTGACCAA CGCCACACCTAACAGCCT GACCATCAGCTGGTACT ACCCCTTCTGCGCCTTCT ACTACCGGATCACCTAT GGCGAGACAGGCGGCAA CAGCCCTAGCCAAGAGT TCACCGTGCCTAGACCT AGCGACACCGCCACAATC TCTGGCCTGAAGCCAGG CCAGGACTACACCATCA CAGTGTACGCCGTGACC TGCCTGGGCAGCTACAG CAGACCCATCAGCATCA ACTACCGGACCGAGATC GACAAGCCCAGCCAA | |
| 21 | Flag-tag | GACTACAAGGACGACGA TGACAAG | Markers to confirm whether antigen-binding domains is expressed extracellularly in non-clinical cell testing stages |
| 22 | CD8 *α* H&TM nucleotide | ACCACCACACCAGCTCCA CGGCCTCCAACACCAGCA CCTACAATTGCCTCTCA GCCCCTGTCTCTGAGGCC AGAAGCTTGTAGACCTG CTGCAGGCGGAGCCGTGC ATACCAGAGGACTGGAT TTCGCCTGCGACATCTA CATTTGGGCCCCTCTGGC TGGAACATGTGGCGTCC TGCTGCTGTCTCTCGTG ATCACCCTGTACTGC | Codon-optimized nucleotide sequence of the Human CD8α hinge and transmembrane domain amino acid sequence (138-206, NCBI accession # NP_001139345.1) |
| 23 | CD28 nucleotide | AGAAGCAAGAGAAGCCG GCTGCTGCACAGCGACT ACATGAACATGACCCCT AGACGGCCCGGACCTACC AGAAAGCACTACCAGCC TTACGCTCCTCCTCGGG ACTTTGCCGCCTATAGA TCC | Optimized nucleotide sequence of the human CD28-derived intracellular signaling domain amino acid sequence (83-123, NCBI accession # NP_001230006.1) |
| 24 | 4-1BB nucleotide | AAGCGGGCAGAAAGAA GCTGCTGTACATCTTCA AGCAGCCCTTCATGCGGC CCGTGCAGACCACACAA GAGGAAGATGGCTGCTC CTGCAGATTCCCCGAGG AAGAAGAAGGCGGCTGC GAGCTG | Optimized nucleotide sequence of the 4-1BB-derived intracellular signaling domain amino acid sequence (214-255, NCBI accession # NP_001552.2) |
| 25 | CD3 ζ nucleotide | AGAGTGAAGTTCTCTAG ATCTGCCGACGCTCCTGC CTACCAGCAGGGACAGA ATCAGCTGTACAACGAG CTGAATCTGGGGCGCAG AGAAGAGTACGACGTGC TGGATAAGAGAAGAGGC AGAGATCCCGAGATGGG CGGCAAGCCCCAGAGAA GAAAGAATCCCCAAGAG GGCCTGTATAATGAGCT GCAGAAAGACAAGATGG CCGAGGCCTACAGCGAG ATCGGAATGAAGGGCGA ACGCAGAAGAGGAAAGG GCCACGACGGACTGTAT CAGGGCCTGAGCACAGCC ACCAAGGATACCTATGA TGCCCTGCACATGCAGGC CCTGCCACCTAGG | Optimized nucleotide sequence of the CD3ζ-derived intracellular signaling domain amino acid sequence (60-172, NCBI accession # XP_011508446.1) |

The nucleic acid sequences of said table 5 are expressed in immune cells, allowing the PD-L1 and hydrophilic EphA2 monobody-based chimeric antigen receptors, consisting of the amino acid sequences of table 6 below, to be expressed on the cell surface membrane.

**[Table 6]**

| SEQ ID NO | Sequence Name | Sequence | Remarks |
|---|---|---|---|
| 26 | CD8 *α* signal sequence amino acid | MALPVTALLLPLALLLHA ARP | Amino acid sequence corresponding to SEQ ID NO: 17 (1-21, NCBI accession # NP_741969.1) |
| 27 | PD-L1 scFv(VYPC) amino acid | QMQLVQSGAEVKKPGSSV KVSCKASGGTFSSYAISW VRQAPGQGLEWMGRIIPIL GIANYAQKFQGRVTITAD KSTSTAYMELSSLRSEDT AVYYCAKPRDGYNLVAFD IWGQGTMVTVSSGLGGLG GGGSGGGGSGGSSGVGSQ LVLTQPPSVSGAPGQRVTI SCTGSSSNIGAGYDVHWY QQLPGAAPKLLIYGDINRP SGVPDRFSGSKSGISASLA ITGLQAEDEADYYCQSYD SSLSGGVFGGGTKLTVL | Amino acid sequence corresponding to SEQ ID NO: 18 |
| 28 | Linker | GGGGSGGGGSGGGGS | Amino acid sequence corresponding to SEQ ID NO: 19 |
| 29 | hpE1 monomer amino acid | SSDSPRNLEVTNATPNSL TISWYYPFCAFYYRITYGE TGGNSPSQEFTVPRPSDT ATISGLKPGQDYTITVYAV TCLGSYSRPISINYRTEIDK PSQ | Amino acid sequence corresponding to SEQ ID NO: 20 |
| 30 | Flag-tag | DYKDDDDK | Amino acid sequence corresponding to SEQ ID NO: 21 |
| 31 | CD8 *α* H&TM amino acid | TTTPAPRPPTPAPTIASQ PLSLRPEACRPAAGGAVH TRGLDFACDIYIWAPLAG TCGVLLLSLVITLYC | Amino acid sequence corresponding to SEQ ID NO: 22 (138-206, NCBI accession# NP_001139345.1) |
| 32 | CD28 amino acid | RSKRSRLLHSDYMNMTPR RPGPTRKHYQPYAPPRDF AAYRS | Amino acid sequence corresponding to SEQ ID NO: 23 (83-123, NCBI accession# NP_001230006.1) |
| 33 | 4-1BB amino acid | KRGRKKLLYIFKQPFMRP VQTTQEEDGCSCRFPEEE EGGCEL | Amino acid sequence corresponding to SEQ ID NO: 24 (214-255, NCBI accession # NP_001552.2) |
| 34 | CD3 ζ amino acid | RVKFSRSADAPAYQQGQN QLYNELNLGRREEYDVLD KRRGRDPEMGGKPQRRKN PQEGLYNELQKDKMAEAY SEIGMKGERRRGKGHDGL YQGLSTATKDTYDALHM QALPPR | Amino acid sequence corresponding to SEQ ID NO: 25 (60-172, NCBI accession# XP_011508446.1) |

### 12-2. Production of recombinant lentiviral vectors expressing PD-L1 single-chain fragment antibody(VYPC)-hydrophilic E1 monobody dual-targeting chimeric antigen receptor

Using said two prepared lentiviral vectors (pLenti7.3::CD8α leader sequence-E1 monobody-CD8 H&T-CD28-CD3ζ vector and pLenti7.3::CD8α leader sequence-El monobody-CD8 H&T-4-1BB-CD3ζ vector) as templates, PCR was performed using the CPL-F primer and CPL-R primer of table 7 below. Two types of monobody-free chimeric antigen receptor vectors were prepared by isolating and purifying the vectors amplified in this manner.

The VYPC ScFv - Linker gene sequence for the dual-targeting chimeric antigen receptor VYPC ScFv - Linker - hpE1 monoobdy - Flag tag (hereinafter referred to as VYPC-Linker-hpE1-Flag) was amplified by performing PCR using said pLenti7.3-VYPC vector with the VYPC-F primer and VYPC-linker-R primer of said table 3. The hpE1 monobody - Flag gene sequence was amplified by performing PCR using the pMX-hpE1 vector with the Linker-hpE1-F primer and hpE1-Flag-R primer of table 7 below. By isolating and purifying said amplified gene sequences, overlap PCR was performed using the VYPC-F primer and hpEl-Flag-R primer of table 7 below. Similar to said method, for hpE1 monobody - Linker - VYPC ScFv - Flag tag (hereinafter referred to as hpE1-Linker-VYPC-Flag), the hpE1-Linker gene sequence was amplified by performing PCR using the pMX-hpE1 vector with the hpE1-F and hpE1-Linker-R primers of table 7 below, and the VYPC-Flag gene sequence was amplified by performing PCR using the pLenti7.3-VYPC vector with the Linker-VYPC-F and VYPC-Flag-R primers of table 7 below. The amplified gene sequences were isolated and purified, and amplified by performing by overlap PCR using the hpE1-F and VYPC-Flag-R primers in Table 3 above.

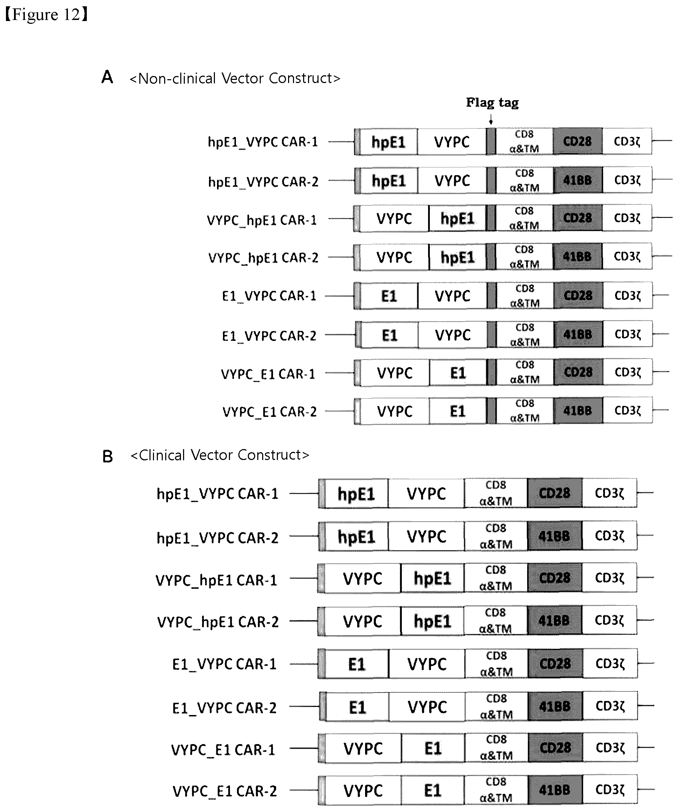

Prepared vectors, and the VYPC-Linker-hpE1-Flag sequence and the hpE1-Linker-VYPC-Flag sequence were inserted into the site where the E1 monobody sequence present through a homologous recombination method using an Overlap cloner DNA cloning kit (Elpisbio), thereby producing four types of recombinant lentiviral vectors (pLenti7.3::CD8α leader -VYPC ScFv - hpE1 monobody chimeric antigen receptor vector and pLenti7.3::CD8α leader - hpE1 monobody - VYPC ScFv chimeric antigen receptor vector) expressing chimeric antigen receptors including dual-targeting antibodies and monobodies. Produced recombinant lentiviral vectors were named PD-L1-hpEphA2 mCAR-1(VYPC-hpVEC-1), PD-Ll-hpEphA2 mCAR-2 (VYPC-hpVEC-2), hpEphA2-PD-L1 mCAR-1(hpVEC-VYPC-1), and hpEphA2-PD-L1 mCAR-2(hpVEC-VYPC-2), and their structures are shown in FIG. 12.

**[Table 7]**

| SEQ ID NO | Sequence Name | Sequence | Remarks |
|---|---|---|---|
| 35 | CPL F | ACCACCACACCAGCTCC | Plasmid linearization and ScFv sequence removal |
| 36 | CPL R | AGGTCTAGCAGCATGCA G | Plasmid linearization and ScFv sequence removal |
| 37 | VYPC-F | CTGCATGCTGCTAGACC TCAGATGCAGCTGGTGC AG | Amplification of VYPC ScFv-linker |
| 38 | VYPC-Linker-R | AGAGCCTCCGCCACCACT TCCTCCGCCGCCAGAACC TCCGCCTCCTAGGACGGT CAGCTTGGTCC | Amplification of VYPC ScFv-linker |
| 39 | Linker-hpE1-F | GGTGGCGGAGGCTCTAG CAGCGACAGCCCCA | Amplification of hpE1-Flag |
| 40 | hpEl-Flag-R | AGCTGGTGTGGTGGTCT TGTCATCGTCGTCCTTG TAGTCTTGGCTGGGCTT GTCGATC | Amplification of hpE1-Flag |
| 41 | hpE1-F | CATGCTGCTAGACCTAG CAGCGACAGCCCC | Amplification of hpE1-Linker |
| 42 | hpE1- Linker-R | AGAGCCTCCGCCACCACT TCCTCCGCCGCCAGAACC TCCGCCTCCTTGGCTGGG CTTGTCGATCTC | Amplification of hpE1-Linker |
| 43 | Linker-VYPC-F | GGTGGCGGAGGCTCTCA GATGCAGCTGGTGCAG | Amplification of VYPC ScFv-Flag |
| 44 | VYPC-Flag-R | AGCTGGTGTGGTGGTCT TGTCATCGTCGTCCTTG TAGTCTAGGACGGTCAG CTTGGTCC | Amplification of VYPC ScFv-Flag |
| 47 | VYPC-hpE1-Flag-CD28 CAR Nucleotide sequence | ATGGCCCTGCCTGTGAC AGCTTTGCTGCTGCCTC TGGCTCTGCTGCTGCAT GCTGCTAGACCTCAGAT GCAGCTGGTGCAGTCTG GGGCTGAGGTGAAGAAG CCTGGGTCCTCGGTGAA GGTCTCCTGCAAGGCTT CTGGAGGCACCTTCAGC AGCTATGCTATCAGCTG GGTGCGACAGGCCCCTGG ACAAGGGCTTGAGTGGA TGGGAAGGATCATCCCT ATCCTTGGTATAGCAAA CTACGCACAGAAGTTCC AGGGCAGAGTCACGATT ACCGCGGACAAATCCAC GAGCACAGCCTACATGG AGCTGAGCAGCCTGAGA TCTGAGGACACGGCCGT GTATTACTGTGCGAAAC CGAGAGATGGCTACAAT TTGGTTGCTTTTGATAT CTGGGGCCAAGGGACGA TGGTCACCGTCTCCTCA GGCCTCGGGGGCCTCGGA GGAGGAGGTAGTGGCGG AGGAGGCTCCGGTGGAT CCAGCGGTGTGGGTTCCC AGCTCGTGCTGACTCAG CCGCCCTCAGTGTCTGGG GCCCCAGGGCAGAGGGTC ACCATCTCCTGCACTGG GAGCAGCTCCAACATCG GGGCAGGTTATGATGTA CACTGGTATCAGCAACT TCCAGGAGCAGCCCCCAA ACTCCTCATCTATGGCG ACATCAATCGGCCCTCA GGGGTCCCTGACCGATT CTCTGGCTCCAAGTCTG GCATCTCAGCCTCCCTGG CTATCACTGGGCTCCAG GCTGAGGACGAGGCTGA TTATTACTGCCAGTCCT ATGACAGCAGCCTGAGT GGGGGGGTGTTCGGCGG AGGGACCAAGCTGACCG TCCTAGGAGGCGGAGGT TCTGGCGGCGGAGGAAG TGGTGGCGGAGGCTCTA GCAGCGACAGCCCAGAA ATCTGGAAGTGACCAAC GCCACACCTAACAGCCTG ACCATCAGCTGGTACTA CCCCTTCTGCGCCTTCTA CTACCGGATCACCTATG GCGAGACAGGCGGCAAC AGCCCTAGCCAAGAGTT CACCGTGCCTAGACCTA GCGACACCGCCACAATCT CTGGCCTGAAGCCAGGCC AGGACTACACCATCACA GTGTACGCCGTGACCTG CCTGGGCAGCTACAGCA GACCCATCAGCATCAAC TACCGGACCGAGATCGA CAAGCCCAGCCAAGACT ACAAGGACGACGATGAC AAGACCACCACACCAGC TCCACGGCCTCCAACACC AGCACCTACAATTGCCT CTCAGCCCCTGTCTCTGA GGCCAGAAGCTTGTAGA CCTGCTGCAGGCGGAGCC GTGCATACCAGAGGACT GGATTTCGCCTGCGACA TCTACATTTGGGCCCCT CTGGCTGGAACATGTGG CGTCCTGCTGCTGTCTC TCGTGATCACCCTGTAC TGCAGAAGCAAGAGAAG CCGGCTGCTGCACAGCGA CTACATGAACATGACCC CTAGACGGCCCGGACCTA CCAGAAAGCACTACCAG CCTTACGCTCCTCCTCGG GACTTTGCCGCCTATAG ATCCAGAGTGAAGTTCT CTAGATCTGCCGACGCT CCTGCCTACCAGCAGGGA CAGAATCAGCTGTACAA CGAGCTGAATCTGGGGC GCAGAGAAGAGTACGAC GTGCTGGATAAGAGAAG AGGCAGAGATCCCGAGA TGGGCGGCAAGCCCCAGA GAAGAAAGAATCCCCAA GAGGGCCTGTATAATGA GCTGCAGAAAGACAAGA TGGCCGAGGCCTACAGCG AGATCGGAATGAAGGGC GAACGCAGAAGAGGAAA GGGCCACGACGGACTGT ATCAGGGCCTGAGCACA GCCACCAAGGATACCTA TGATGCCCTGCACATGC AGGCCCTGCCACCTAGG | |
| 48 | hpE1-VYPC-Flag-CD28 CAR Nucleotide sequence | ATGGCCCTGCCTGTGAC AGCTTTGCTGCTGCCTC TGGCTCTGCTGCTGCAT GCTGCTAGACCTAGCAG CGACAGCCCCAGAAATC TGGAAGTGACCAACGCC ACACCTAACAGCCTGAC CATCAGCTGGTACTACC CCTTCTGCGCCTTCTAC TACCGGATCACCTATGG CGAGACAGGCGGCAACA GCCCTAGCCAAGAGTTC ACCGTGCCTAGACCTAG CGACACCGCCACAATCTC TGGCCTGAAGCCAGGCC AGGACTACACCATCACA GTGTACGCCGTGACCTG CCTGGGCAGCTACAGCA GACCCATCAGCATCAAC TACCGGACCGAGATCGA CAAGCCCAGCCAAGGAG GCGGAGGTTCTGGCGGC GGAGGAAGTGGTGGCGG AGGCTCTCAGATGCAGC TGGTGCAGTCTGGGGCT GAGGTGAAGAAGCCTGG GTCCTCGGTGAAGGTCT CCTGCAAGGCTTCTGGA GGCACCTTCAGCAGCTA TGCTATCAGCTGGGTGC GACAGGCCCCTGGACAA GGGCTTGAGTGGATGGG AAGGATCATCCCTATCC TTGGTATAGCAAACTAC GCACAGAAGTTCCAGGG CAGAGTCACGATTACCG CGGACAAATCCACGAGC ACAGCCTACATGGAGCT GAGCAGCCTGAGATCTG AGGACACGGCCGTGTAT TACTGTGCGAAACCGAG AGATGGCTACAATTTGG TTGCTTTTGATATCTGG GGCCAAGGGACGATGGT CACCGTCTCCTCAGGCCT CGGGGGCCTCGGAGGAGG AGGTAGTGGCGGAGGAG GCTCCGGTGGATCCAGCG GTGTGGGTTCCCAGCTC GTGCTGACTCAGCCGCCC TCAGTGTCTGGGGCCCC AGGGCAGAGGGTCACCA TCTCCTGCACTGGGAGC AGCTCCAACATCGGGGC AGGTTATGATGTACACT GGTATCAGCAACTTCCA GGAGCAGCCCCCAAACTC CTCATCTATGGCGACAT CAATCGGCCCTCAGGGG TCCCTGACCGATTCTCT GGCTCCAAGTCTGGCAT CTCAGCCTCCCTGGCTAT CACTGGGCTCCAGGCTG AGGACGAGGCTGATTAT TACTGCCAGTCCTATGA CAGCAGCCTGAGTGGGG GGGTGTTCGGCGGAGGG ACCAAGCTGACCGTCCT AGACTACAAGGACGACG ATGACAAGACCACCACA CCAGCTCCACGGCCTCCA ACACCAGCACCTACAAT TGCCTCTCAGCCCCTGTC TCTGAGGCCAGAAGCTT GTAGACCTGCTGCAGGC GGAGCCGTGCATACCAG AGGACTGGATTTCGCCT GCGACATCTACATTTGG GCCCCTCTGGCTGGAACA TGTGGCGTCCTGCTGCT GTCTCTCGTGATCACCC TGTACTGCAGAAGCAAG AGAAGCCGGCTGCTGCA CAGCGACTACATGAACA TGACCCCTAGACGGCCCG GACCTACCAGAAAGCAC TACCAGCCTTACGCTCC TCCTCGGGACTTTGCCGC CTATAGATCCAGAGTGA AGTTCTCTAGATCTGCC GACGCTCCTGCCTACCAG CAGGGACAGAATCAGCT GTACAACGAGCTGAATC TGGGGCGCAGAGAAGAG TACGACGTGCTGGATAA GAGAAGAGGCAGAGATC CCGAGATGGGCGGCAAG CCCCAGAGAAGAAAGAA TCCCCAAGAGGGCCTGT ATAATGAGCTGCAGAAA GACAAGATGGCCGAGGC CTACAGCGAGATCGGAA TGAAGGGCGAACGCAGA AGAGGAAAGGGCCACGA CGGACTGTATCAGGGCC TGAGCACAGCCACCAAG GATACCTATGATGCCCT GCACATGCAGGCCCTGCC ACCTAGG | |
| 49 | hpE1 monobody-CD28 CAR Nucleotide sequence | ATGGCCCTGCCTGTGAC AGCTTTGCTGCTGCCTC TGGCTCTGCTGCTGCAT GCTGCTAGACCTAGCAG CGACAGCCCCAGAAATC TGGAAGTGACCAACGCC ACACCTAACAGCCTGAC CATCAGCTGGTACTACC CCTTCTGCGCCTTCTAC TACCGGATCACCTATGG CGAGACAGGCGGCAACA GCCCTAGCCAAGAGTTC ACCGTGCCTAGACCTAG CGACACCGCCACAATCTC TGGCCTGAAGCCAGGCC AGGACTACACCATCACA GTGTACGCCGTGACCTG CCTGGGCAGCTACAGCA GACCCATCAGCATCAAC TACCGGACCGAGATCGA CAAGCCCAGCCAAACCAC CACACCAGCTCCACGGCC TCCAACACCAGCACCTAC AATTGCCTCTCAGCCCC TGTCTCTGAGGCCAGAA GCTTGTAGACCTGCTGC AGGCGGAGCCGTGCATA CCAGAGGACTGGATTTC GCCTGCGACATCTACAT TTGGGCCCCTCTGGCTGG AACATGTGGCGTCCTGC TGCTGTCTCTCGTGATC ACCCTGTACTGCAGAAG CAAGAGAAGCCGGCTGC TGCACAGCGACTACATG AACATGACCCCTAGACG GCCCGGACCTACCAGAA AGCACTACCAGCCTTAC GCTCCTCCTCGGGACTT TGCCGCCTATAGATCCA GAGTGAAGTTCTCTAGA TCTGCCGACGCTCCTGCC TACCAGCAGGGACAGAA TCAGCTGTACAACGAGC TGAATCTGGGGCGCAGA GAAGAGTACGACGTGCT GGATAAGAGAAGAGGCA GAGATCCCGAGATGGGC GGCAAGCCCCAGAGAAG AAAGAATCCCCAAGAGG GCCTGTATAATGAGCTG CAGAAAGACAAGATGGC CGAGGCCTACAGCGAGA TCGGAATGAAGGGCGAA CGCAGAAGAGGAAAGGG CCACGACGGACTGTATC AGGGCCTGAGCACAGCC ACCAAGGATACCTATGA TGCCCTGCACATGCAGGC CCTGCCACCTAGG | |
| 50 | hpE1 monobody-41BB CAR Nucleotide sequence | ATGGCCCTGCCTGTGAC AGCTTTGCTGCTGCCTC TGGCTCTGCTGCTGCAT GCTGCTAGACCTAGCAG CGACAGCCCCAGAAATC TGGAAGTGACCAACGCC ACACCTAACAGCCTGAC CATCAGCTGGTACTACC CCTTCTGCGCCTTCTAC TACCGGATCACCTATGG CGAGACAGGCGGCAACA GCCCTAGCCAAGAGTTC ACCGTGCCTAGACCTAG CGACACCGCCACAATCTC TGGCCTGAAGCCAGGCC AGGACTACACCATCACA GTGTACGCCGTGACCTG CCTGGGCAGCTACAGCA GACCCATCAGCATCAAC TACCGGACCGAGATCGA CAAGCCCAGCCAAACCAC CACACCAGCTCCACGGCC TCCAACACCAGCACCTAC AATTGCCTCTCAGCCCC TGTCTCTGAGGCCAGAA GCTTGTAGACCTGCTGC AGGCGGAGCCGTGCATA CCAGAGGACTGGATTTC GCCTGCGACATCTACAT TTGGGCCCCTCTGGCTGG AACATGTGGCGTCCTGC TGCTGTCTCTCGTGATC ACCCTGTACTGCAAGCG GGGCAGAAAGAAGCTGC TGTACATCTTCAAGCAG CCCTTCATGCGGCCCGTG CAGACCACACAAGAGGA AGATGGCTGCTCCTGCA GATTCCCCGAGGAAGAA GAAGGCGGCTGCGAGCT GAGAGTGAAGTTCTCTA GATCTGCCGACGCTCCTG CCTACCAGCAGGGACAG AATCAGCTGTACAACGA GCTGAATCTGGGGCGCA GAGAAGAGTACGACGTG CTGGATAAGAGAAGAGG CAGAGATCCCGAGATGG GCGGCAAGCCCCAGAGA AGAAAGAATCCCCAAGA GGGCCTGTATAATGAGC TGCAGAAAGACAAGATG GCCGAGGCCTACAGCGAG ATCGGAATGAAGGGCGA ACGCAGAAGAGGAAAGG GCCACGACGGACTGTAT CAGGGCCTGAGCACAGCC ACCAAGGATACCTATGA TGCCCTGCACATGCAGGC CCTGCCACCTAGG | |
| 51 | VYPC-hpE1-Flag-41BB CAR nucleotide sequence | ATGGCCCTGCCTGTGAC AGCTTTGCTGCTGCCTC TGGCTCTGCTGCTGCAT GCTGCTAGACCTCAGAT GCAGCTGGTGCAGTCTG GGGCTGAGGTGAAGAAG CCTGGGTCCTCGGTGAA GGTCTCCTGCAAGGCTT CTGGAGGCACCTTCAGC AGCTATGCTATCAGCTG GGTGCGACAGGCCCCTGG ACAAGGGCTTGAGTGGA TGGGAAGGATCATCCCT ATCCTTGGTATAGCAAA CTACGCACAGAAGTTCC AGGGCAGAGTCACGATT ACCGCGGACAAATCCAC GAGCACAGCCTACATGG AGCTGAGCAGCCTGAGA TCTGAGGACACGGCCGT GTATTACTGTGCGAAAC CGAGAGATGGCTACAAT TTGGTTGCTTTTGATAT CTGGGGCCAAGGGACGA TGGTCACCGTCTCCTCA GGCCTCGGGGGCCTCGGA GGAGGAGGTAGTGGCGG AGGAGGCTCCGGTGGAT CCAGCGGTGTGGGTTCCC AGCTCGTGCTGACTCAG CCGCCCTCAGTGTCTGGG GCCCCAGGGCAGAGGGTC ACCATCTCCTGCACTGG GAGCAGCTCCAACATCG GGGCAGGTTATGATGTA CACTGGTATCAGCAACT TCCAGGAGCAGCCCCCAA ACTCCTCATCTATGGCG ACATCAATCGGCCCTCA GGGGTCCCTGACCGATT CTCTGGCTCCAAGTCTG GCATCTCAGCCTCCCTGG CTATCACTGGGCTCCAG GCTGAGGACGAGGCTGA TTATTACTGCCAGTCCT ATGACAGCAGCCTGAGT GGGGGGTGTTCGGCGG AGGGACCAAGCTGACCG TCCTAGGAGGCGGAGGT TCTGGCGGCGGAGGAAG TGGTGGCGGAGGCTCTA GCAGCGACAGCCCCAGAA ATCTGGAAGTGACCAAC GCCACACCTAACAGCCTG ACCATCAGCTGGTACTA CCCCTTCTGCGCCTTCTA CTACCGGATCACCTATG GCGAGACAGGCGGCAAC AGCCCTAGCCAAGAGTT CACCGTGCCTAGACCTA GCGACACCGCCACAATCT CTGGCCTGAAGCCAGGCC AGGACTACACCATCACA GTGTACGCCGTGACCTG CCTGGGCAGCTACAGCA GACCCATCAGCATCAAC TACCGGACCGAGATCGA CAAGCCCAGCCAAGACT ACAAGGACGACGATGAC AAGACCACCACACCAGC TCCACGGCCTCCAACACC AGCACCTACAATTGCCT CTCAGCCCCCTGTCTCTGA GGCCAGAAGCTTGTAGA CCTGCTGCAGGCGGAGCC GTGCATACCAGAGGACT GGATTTCGCCTGCGACA TCTACATTTGGGCCCCT CTGGCTGGAACATGTGG CGTCCTGCTGCTGTCTC TCGTGATCACCCTGTAC TGCAAGCGGGGCAGAAA GAAGCTGCTGTACATCT TCAAGCAGCCCTTCATG CGGCCCGTGCAGACCACA CAAGAGGAAGATGGCTG CTCCTGCAGATTCCCCGA GGAAGAAGAAGGCGGCT GCGAGCTGAGAGTGAAG TTCTCTAGATCTGCCGA CGCTCCTGCCTACCAGCA GGGACAGAATCAGCTGT ACAACGAGCTGAATCTG GGGCGCAGAGAAGAGTA CGACGTGCTGGATAAGA GAAGAGGCAGAGATCCC GAGATGGGCGGCAAGCC CCAGAGAAGAAAGAATC CCCAAGAGGGCCTGTAT AATGAGCTGCAGAAAGA CAAGATGGCCGAGGCCT ACAGCGAGATCGGAATG AAGGGCGAACGCAGAAG AGGAAAGGGCCACGACG GACTGTATCAGGGCCTG AGCACAGCCACCAAGGA TACCTATGATGCCCTGC ACATGCAGGCCCTGCCAC CTAGG | |
| 52 | hpE1-VYPC-Flag-41BB CAR nucleotide sequence | ATGGCCCTGCCTGTGAC AGCTTTGCTGCTGCCTC TGGCTCTGCTGCTGCAT GCTGCTAGACCTAGCAG CGACAGCCCCAGAAATC TGGAAGTGACCAACGCC ACACCTAACAGCCTGAC CATCAGCTGGTACTACC CCTTCTGCGCCTTCTAC TACCGGATCACCTATGG CGAGACAGGCGGCAACA GCCCTAGCCAAGAGTTC ACCGTGCCTAGACCTAG CGACACCGCCACAATCTC TGGCCTGAAGCCAGGCC AGGACTACACCATCACA GTGTACGCCGTGACCTG CCTGGGCAGCTACAGCA GACCCATCAGCATCAAC TACCGGACCGAGATCGA CAAGCCCAGCCAAGGAG GCGGAGGTTCTGGCGGC GGAGGAAGTGGTGGCGG AGGCTCTCAGATGCAGC TGGTGCAGTCTGGGGCT GAGGTGAAGAAGCCTGG GTCCTCGGTGAAGGTCT CCTGCAAGGCTTCTGGA GGCACCTTCAGCAGCTA TGCTATCAGCTGGGTGC GACAGGCCCCTGGACAA GGGCTTGAGTGGATGGG AAGGATCATCCCTATCC TTGGTATAGCAAACTAC GCACAGAAGTTCCAGGG CAGAGTCACGATTACCG CGGACAAATCCACGAGC ACAGCCTACATGGAGCT GAGCAGCCTGAGATCTG AGGACACGGCCGTGTAT TACTGTGCGAAACCGAG AGATGGCTACAATTTGG TTGCTTTTGATATCTGG GGCCAAGGGACGATGGT CACCGTCTCCTCAGGCCT CGGGGGCCTCGGAGGAGG AGGTAGTGGCGGAGGAG GCTCCGGTGGATCCAGCG GTGTGGGTTCCCAGCTC GTGCTGACTCAGCCGCCC TCAGTGTCTGGGGCCCC AGGGCAGAGGGTCACCA TCTCCTGCACTGGGAGC AGCTCCAACATCGGGGC AGGTTATGATGTACACT GGTATCAGCAACTTCCA GGAGCAGCCCCCAAACTC CTCATCTATGGCGACAT CAATCGGCCCTCAGGGG TCCCTGACCGATTCTCT GGCTCCAAGTCTGGCAT CTCAGCCTCCCTGGCTAT CACTGGGCTCCAGGCTG AGGACGAGGCTGATTAT TACTGCCAGTCCTATGA CAGCAGCCTGAGTGGG GGGTGTTCGGCGGAGGG ACCAAGCTGACCGTCCT AGACTACAAGGACGACG ATGACAAGACCACCACA CCAGCTCCACGGCCTCCA ACACCAGCACCTACAAT TGCCTCTCAGCCCCTGTC TCTGAGGCCAGAAGCTT GTAGACCTGCTGCAGGC GGAGCCGTGCATACCAG AGGACTGGATTTCGCCT GCGACATCTACATTTGG GCCCCTCTGGCTGGAACA TGTGGCGTCCTGCTGCT GTCTCTCGTGATCACCC TGTACTGCAAGCGGGGC AGAAAGAAGCTGCTGTA CATCTTCAAGCAGCCCT TCATGCGGCCCGTGCAG ACCACACAAGAGGAAGA TGGCTGCTCCTGCAGAT TCCCCGAGGAAGAAGAA GGCGGCTGCGAGCTGAG AGTGAAGTTCTCTAGAT CTGCCGACGCTCCTGCCT ACCAGCAGGGACAGAAT CAGCTGTACAACGAGCT GAATCTGGGGCGCAGAG AAGAGTACGACGTGCTG GATAAGAGAAGAGGCAG AGATCCCGAGATGGGCG GCAAGCCCCAGAGAAGA AAGAATCCCCAAGAGGG CCTGTATAATGAGCTGC AGAAAGACAAGATGGCC GAGGCCTACAGCGAGAT CGGAATGAAGGGCGAAC GCAGAAGAGGAAAGGGC CACGACGGACTGTATCA GGGCCTGAGCACAGCCAC CAAGGATACCTATGATG CCCTGCACATGCAGGCCC TGCCACCTAGG | |
| 53 | VYPC-hpE1-Flag-CD28 CAR amino acid sequence | MALPVTALLLPLALLLHA ARPQMQLVQSGAEVKKPG SSVKVSCKASGGTFSSYAI SWVRQAPGQGLEWMGRII PILGIANYAQKFQGRVTIT ADKSTSTAYMELSSLRSE DTAVYYCAKPRDGYNLVA FDIWGQGTMVTVSSGLGG LGGGGSGGGGSGGSSGVG SQLVLTQPPSVSGAPGQR VTISCTGSSSNIGAGYDVH WYQQLPGAAPKLLIYGDIN RPSGVPDRFSGSKSGISAS LAITGLQAEDEADYYCQS YDSSLSGGVFGGGTKLTV LGGGGSGGGGSGGGGSSS DSPRNLEVTNATPNSLTI SWYYPFCAFYYRITYGET GGNSPSQEFTVPRPSDTA TISGLKPGQDYTITVYAVT CLGSYSRPISINYRTEIDKP SQDYKDDDDKTTTPAPRP PTPAPTIASQPLSLRPEAC RPAAGGAVHTRGLDFACD IYIWAPLAGTCGVLLLSLV ITLYCRSKRSRLLHSDYMN MTPRRPGPTRKHYQPYAP PRDFAAYRSRVKFSRSAD APAYQQGQNQLYNELNLG RREEYDVLDKRRGRDPEM GGKPQRRKNPQEGLYNEL QKDKMAEAYSEIGMKGER RRGKGHDGLYQGLSTATK DTYDALHMQALPPR | |
| 54 | hpE1-VYPC-Flag-CD28 CAR amino acid sequence | MALPVTALLLPLALLLHA ARPSSDSPRNLEVTNATP NSLTISWYYPFCAFYYRIT YGETGGNSPSQEFTVPRP SDTATISGLKPGQDYTITV YAVTCLGSYSRPISINYRT EIDKPSQGGGGSGGGGSG GGGSQMQLVQSGAEVKKP GSSVKVSCKASGGTFSSY AISWVRQAPGQGLEWMG RIIPILGIANYAQKFQGRVT ITADKSTSTAYMELSSLR SEDTAVYYCAKPRDGYNL VAFDIWGQGTMVTVSSGL GGLGGGGSGGGGSGGSSG VGSQLVLTQPPSVSGAPG QRVTISCTGSSSNIGAGYD VHWYQQLPGAAPKLLIYG DINRPSGVPDRFSGSKSGI SASLAITGLQAEDEADYY CQSYDSSLSGGVFGGGTK LTVLDYKDDDDKTTTPAP RPPTPAPTIASQPLSLRPE ACRPAAGGAVHTRGLDFA CDIYIWAPLAGTCGVLLLS LVITLYCRSKRSRLLHSDY MNMTPRRPGPTRKHYQP YAPPRDFAAYRSRVKFSR SADAPAYQQGQNQLYNEL NLGRREEYDVLDKRRGRD PEMGGKPQRRKNPQEGLY NELQKDKMAEAYSEIGMK GERRRGKGHDGLYQGLST ATKDTYDALHMQALPPR | |
| 55 | hpE1 monobody-CD28 CAR amino acid sequence | MALPVTALLLPLALLLHA ARPSSDSPRNLEVTNATP NSLTISWYYPFCAFYYRIT YGETGGNSPSQEFTVPRP SDTATISGLKPGQDYTITV YAVTCLGSYSRPISINYRT EIDKPSQTTTPAPRPPTP APTIASQPLSLRPEACRPA AGGAVHTRGLDFACDIYI WAPLAGTCGVLLLSLVIT LYCRSKRSRLLHSDYMNM TPRRPGPTRKHYQPYAPP RDFAAYRSRVKFSRSADA PAYQQGQNQLYNELNLGR REEYDVLDKRRGRDPEMG GKPQRRKNPQEGLYNELQ KDKMAEAYSEIGMKGERR RGKGHDGLYQGLSTATKD TYDALHMQALPPR | |
| 56 | hpE1 monobody - | MALPVTALLLPLALLLHA | |
| | 41BB CAR amino acid sequence | ARPSSDSPRNLEVTNATP NSLTISWYYPFCAFYYRIT YGETGGNSPSQEFTVPRP SDTATISGLKPGQDYTITV YAVTCLGSYSRPISINYRT EIDKPSQTTTPAPRPPTP APTIASQPLSLRPEACRPA AGGAVHTRGLDFACDIYI WAPLAGTCGVLLLSLVIT LYCKRGRKKLLYIFKQPF MRPVQTTQEEDGCSCRFP EEEEGGCELRVKFSRSAD APAYQQGQNQLYNELNLG RREEYDVLDKRRGRDPEM GGKPQRRKNPQEGLYNEL QKDKMAEAYSEIGMKGER RRGKGHDGLYQGLSTATK DTYDALHMQALPPR | |
| *57* | VYPC-hpE1-Flag-41BB CAR amino acid sequence | MALPVTALLLPLALLLHA ARPQMQLVQSGAEVKKPG SSVKVSCKASGGTFSSYAI SWVRQAPGQGLEWMGRII PILGIANYAQKFQGRVTIT ADKSTSTAYMELSSLRSE DTAVYYCAKPRDGYNLVA FDIWGQGTMVTVSSGLGG LGGGGSGGGGSGGSSGVG SQLVLTQPPSVSGAPGQR VTISCTGSSSNIGAGYDVH WYQQLPGAAPKLLIYGDIN RPSGVPDRFSGSKSGISAS LAITGLQAEDEADYYCQS YDSSLSGGVFGGGTKLTV LGGGGSGGGGSGGGGSSS DSPRNLEVTNATPNSLTI SWYYPFCAFYYRITYGET GGNSPSQEFTVPRPSDTA TISGLKPGQDYTITVYAVT CLGSYSRPISINYRTEIDKP SQDYKDDDDKTTTPAPRP PTPAPTIASQPLSLRPEAC RPAAGGAVHTRGLDFACD IYIWAPLAGTCGVLLLSLV ITLYCKRGRKKLLYIFKQP FMRPVQTTQEEDGCSCRF PEEEEGGCELRVKFSRSA DAPAYQQGQNQLYNELNL GRREEYDVLDKRRGRDPE MGGKPQRRKNPQEGLYNE LQKDKMAEAYSEIGMKGE RRRGKGHDGLYQGLSTAT KDTYDALHMQALPPR | |
| 58 | hpE1-VYPC-Flag-41BB CAR amino acid sequence | MALPVTALLLPLALLLHA ARPSSDSPRNLEVTNATP NSLTISWYYPFCAFYYRIT YGETGGNSPSQEFTVPRP SDTATISGLKPGQDYTITV YAVTCLGSYSRPISINYRT EIDKPSQGGGGSGGGGSG GGGSQMQLVQSGAEVKKP GSSVKVSCKASGGTFSSY AISWVRQAPGQGLEWMG RIIPILGIANYAQKFQGRVT ITADKSTSTAYMELSSLR SEDTAVYYCAKPRDGYNL VAFDIWGQGTMVTVSSGL GGLGGGGSGGGGSGGSSG VGSQLVLTQPPSVSGAPG QRVTISCTGSSSNIGAGYD VHWYQQLPGAAPKLLIYG DINRPSGVPDRFSGSKSGI SASLAITGLQAEDEADYY CQSYDSSLSGGVFGGGTK LTVLDYKDDDDKTTTPAP RPPTPAPTIASQPLSLRPE ACRPAAGGAVHTRGLDFA CDIYIWAPLAGTCGVLLLS LVITLYCKRGRKKLLYIFK QPFMRPVQTTQEEDGCSC RFPEEEEGGCELRVKFSRS ADAPAYQQGQNQLYNELN LGRREEYDVLDKRRGRDP EMGGKPQRRKNPQEGLYN ELQKDKMAEAYSEIGMKG ERRRGKGHDGLYQGLSTA TKDTYDALHMQALPPR | |

### [Example 13]

### Comparison and Evaluation of Activities of VYPC, hpVEC-1, and Dual-Targeting CAR Cells Including them

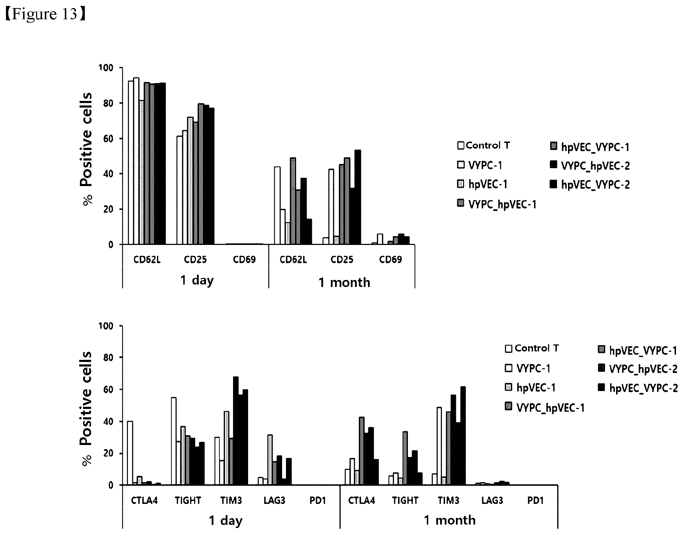

The expression of activation and inhibitory factors in the produced dual chimeric antigen receptor T cells was compared with single chimeric antigen receptor T cells over time. As shown in FIG. 13, immediately after production, CD62L showed a high expression rate in all; however, when compared one month later, CD62L expression decreased in all, and it was particularly observed that it remained higher in the dual CAR-T cells compared to the two single CAR-T cells. Similarly, it was observed that CD25 was generally expressed at higher levels in the dual CAR-T cells compared to the single CAR-T cells. In the case of inhibitory factors, immediately after production, CTLA-4 and PD-1 were hardly expressed, and TIGIT was expressed at almost similar levels. Except for the dual CAR-T expressing VYPC_hpVEC-1, all showed higher expression of TIM-3 compared to the single CAR-T. One month later, CTLA-4, TIGIT, and TIM-3 all tended to be expressed at higher levels in the dual CAR-T.

### [Example 14]

### Comparison of In Vitro Cytotoxicity of Dual-Targeting CAR Cells Including PD-L1 scFv and Hydrophilic EphA2 Monobody in Antigen PD-L1 and EphA2-Overexpressing Cell Lines

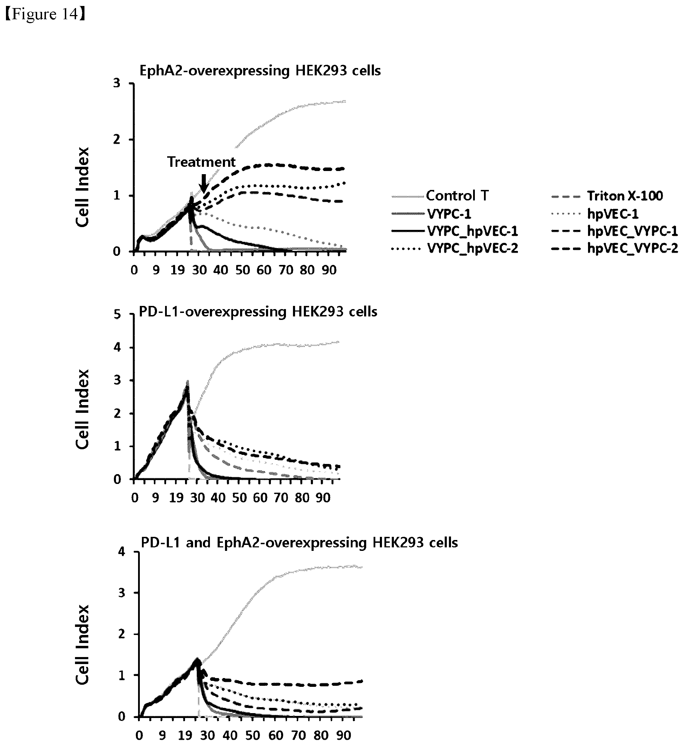

Investigation was conducted to determine whether the produced dual-targeting CAR-T has cytotoxicity under conditions where HEK293 cells expressing PD-L1, HEK293 cells expressing EphA2, and cells expressing both PD-1 and EphA2 are mixed. To produce HEK293 cells expressing PD-L1, a plasmid in which EphA2 cDNA is cloned was purchased from addgene (#121486) and introduced into HEK293 cells using lipofectamine2000. After 48 hours, treatment with puromycin 5 ug/ml was performed to select only the cells in which the plasmid was inserted into the HEK293 cell genome, and then single cells were transferred one by one to a 96-well plate and cultured. Each clone proliferated from a single cell was confirmed for the expression of EphA2 protein in HEK293 cells through FACS analysis using PD-L1-PE antibody. PD-L1-overexpressing HEK293 cells and EphA2-overexpressing HEK293 cells were cultured in an RTCA plate at 1 x 10⁴/100 ul each, and PD-L1-overexpressing cells and EphA2-overexpressing cells were mixed at 0.5 x 10⁴ cells each and cultured; then, after 24 hours, single CAR-T and dual CAR-T were treated at 1 x 10^/100 ul each, followed by RTCA analysis. As a result of the analysis, as shown in FIG. 14, it was confirmed that VYPC_hpVEC-1 has significantly higher anticancer activity than hpVEC-1.

### [Example 15]

### Comparison of Anticancer Activity of Dual-Targeting CAR Cells Including PD-L1 scFv and Hydrophilic EphA2 Monobody Against Two Subtypes of OVCARS Derived from Ovarian Cancer Metastasized from Gastric Cancer

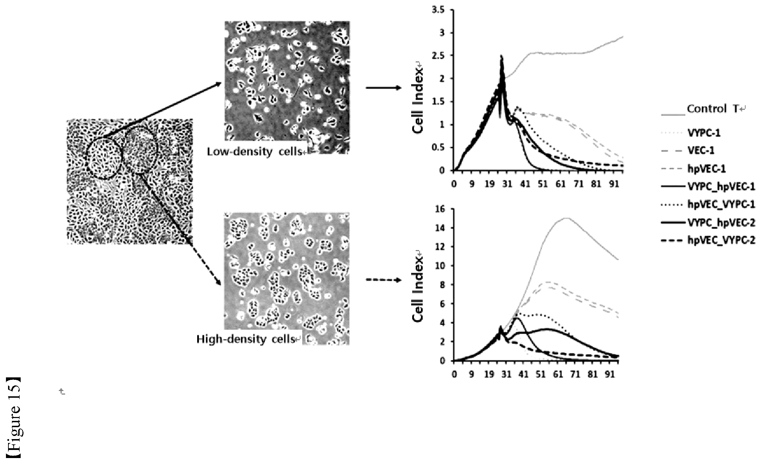

To verify the efficacy of the produced dual CAR-T in cancer cell lines and compare it with single CAR-T, RTCA was performed using an ovarian cancer cell line (OVCAR5) in the same manner as the method mentioned above. OVCAR5 cells were cultured in a microtiter plate at 1 x 10⁴/100 ul each, and after 24 hours, each single and dual CAR-T was treated at 1 x 10⁴/100 ul each, followed by monitoring for 72 hours. As a result of the test, as shown in FIG. 15, the VYPC hpVEC-1 or hpVEC_VYPC-2 dual-targeting immune cells showed significantly superior cytotoxicity compared to the VEC-1 and hpVEC-1 single-targeting immune cells.

### [Example 16]

### Evaluation of Anti-tumor Activity of Single-Targeting and Dual-Targeting Immune Cells in a Xenograft Mouse Model

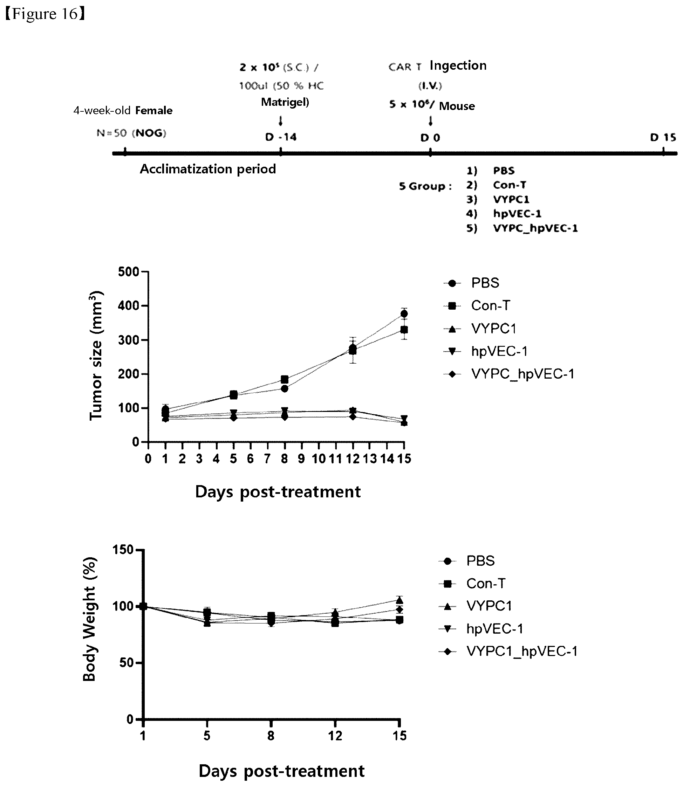

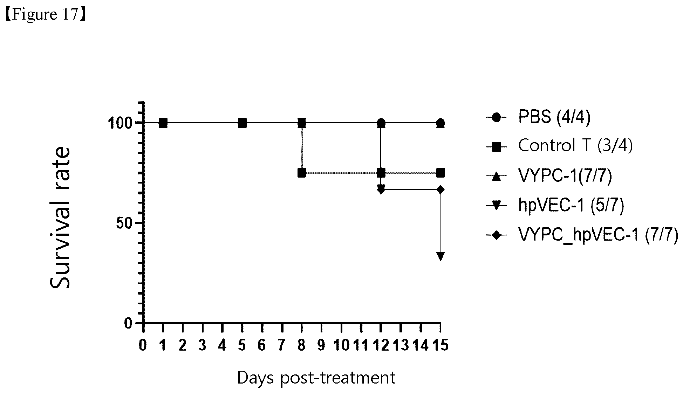

Animal tests were performed in the same manner as Example 11, and as a result of the tests, as shown in FIGs. 16 and 17, in the ovarian cancer cell line OVCAR-5 xenograft disease model, single-targeting(hpVEC-1, VYPC-1) and VYPC_hpVEC-1 dual-targeting CAR cells all showed a similarly high level of cytotoxicity; however, in terms of toxicity, toxicity was not observed in VYPC-1 and VYPC_hpVEC-1, and the body weight recovery speed of mice was fast and good. On the other hand, the mice injected with hpVEC-1 showed a continuous decrease in body weight and a loss of hair gloss, and 2 out of 7 mice died at 15 days, the end point of the experiment.

It can be seen that the VYPC_hpVEC-1 monobody-based dual-targeting chimeric antigen receptor according to the present invention and immune cells (e.g., cytotoxic T cells) expressing the same on the cell membrane surface have excellent anticancer effects by significantly inhibiting the proliferation of cancer cells such as ovarian cancer and gastric cancer and significantly increasing the survival rate of mice, and it was confirmed that the toxicity issue of the hpVEC-1 single-targeting is remarkably improved. Therefore, solid tumors can be prevented or treated using immune cells including the PD-L1 scFv and hydrophilic monobody-based chimeric antigen receptor.

## Claims

1. A monobody that specifically binds to EphA2, wherein one or more hydrophobic amino acids of the monobody consisting of the amino acid sequence of SEQ ID NO: 46 are substituted with hydrophilic amino acids and/or one or more hydrophilic amino acids are added to the same.

2. The monobody of claim 1, comprising the amino acid sequence of SEQ ID NO: 8.

3. The monobody of claim 1, which is connected to PD-L1 scFv directly or through a linker.

4. A chimeric antigen receptor comprising the monobody according to any one of claims 1 to 3 as an extracellular ligand-binding domain, and further comprising one or more selected from the group consisting of a transmembrane domain and an intracellular signaling domain.

5. The chimeric antigen receptor of claim 4, wherein said transmembrane domain is selected from the group consisting of T-cell receptor alpha chain, T-cell receptor beta chain, T-cell receptor zeta chain, CD8 alpha chain, CD8 beta chain, CD28, CD3 epsilon, CD45, CD4, CD5, CD8, CD9, CD16, CD22, CD33, CD37, CD64, CD80, CD86, CD134, CD137, CD154, a part thereof, and any combination thereof.

6. The chimeric antigen receptor of claim 4, wherein said intracellular signaling domain is selected from the group consisting of TCR zeta, FcR gamma, FcR beta, FcR epsilon, CD3 gamma, CD3 delta, CD3 epsilon, CD3 zeta, CD5, CD22, CD79a, CD79b, CD66d, and any combination thereof.

7. The chimeric antigen receptor of claim 4, wherein said intracellular signaling domain is one or more selected from the group consisting of OX40 domain, CD2 domain, CD27 domain, CD28 domain, CDS domain, ICAM-1 domain, LFA-1 domain, 4-1BB domain, and any combination thereof.

8. The chimeric antigen receptor of claim 4, further comprising a hinge.

9. A polynucleotide comprising a nucleic acid sequence encoding the monobody according to any one of claims 1 to 3 or the chimeric antigen receptor according to any one of claims 4 to 8.

10. A vector comprising the polynucleotide according to claim 9.

11. An isolated immune cell expressing, on its cell surface membrane, an extracellular ligand-binding domain comprising the monobody according to any one of claims 1 to 3 or the chimeric antigen receptor according to any one of claims 4 to 8.

12. The isolated immune cell of claim 11, which is selected from the group consisting of a leukocyte, a neutrophil, an eosinophil, a basophil, a monocyte, a lymphocyte, a T cell, a cytotoxic T cell, a natural killer T cell, and a dendritic cell.

13. A pharmaceutical composition for preventing or treating cancer, comprising the isolated immune cell according to claim 11.

14. The pharmaceutical composition of claim 13, wherein said cancer is one or more selected from the group consisting of melanoma, squamous cell carcinoma, breast cancer, head and neck cancer, thyroid cancer, soft tissue sarcoma, osteosarcoma, testicular cancer, prostate cancer, ovarian cancer, bladder cancer, skin cancer, brain cancer, angiosarcoma, mastocytoma, leukemia, lymphoma, liver cancer, lung cancer, pancreatic cancer, gastric cancer, kidney cancer, colorectal cancer, hematopoietic tumor, and metastatic cancer thereof.

15. The pharmaceutical composition of claim 14, wherein said cancer is pancreatic cancer, prostate cancer, or ovarian cancer.
